# EUROPÄISCHE PATENTSCHRIFT

(11) **EP 2 428 792 B1**
(45) Veröffentlichungstag und Bekanntmachung des Hinweises auf die Patenterteilung: **08.10.2014**
(21) Anmeldenummer: 11180576.8
(22) Anmeldetag: 08.09.2011
(51) Int. Cl.: G01N 21/25, G01N 21/76, G01N 21/64, C12M 1/34, B01L 5/00, B01L 3/00

(54) **Mikroplatten-Reader mit kontrollierter Gasatmosphäre, entsprechendes Verfahren und Verwendung derselben**
Microplate reader with controlled gas atmosphere, corresponding method and use of the same
Lecteur à microplaquettes doté d'une atmosphère gazeuse contrôlée, procédé correspondant et utilisation de celui-ci

(30) Priorität: 08.09.2010 CH 14462010
(43) Veröffentlichungstag der Anmeldung: 14.03.2012
(73) Patentinhaber: Tecan Trading AG, 8708 Männedorf (CH)
(72) Erfinder: Probst, Gerald, 5071 Wals (AT); Schürf, Markus, 83346 Bergen / Bernhaupten (DE); Grassl, Josef, 83471 Schönau am Königsee (DE)
(74) Vertreter: OK pat AG

(56) Entgegenhaltungen:
- EP-A1- 2 325 326
- WO-A1-2010/010670
- DE-A1-102005 033 927
- US-A1- 2005 196 325
- US-A1- 2006 023 299
- US-A1- 2008 180 793
- US-B1- 6 548 263

## Beschreibung

Die Erfindung betrifft einen Mikroplatten-Reader, der zumindest eine Messeinrichtung und eine Aufnahmeeinrichtung umfasst. Die zumindest eine Messeinrichtung dient zum Detektieren von Licht, das von Proben in Wells einer in diesen Mikroplatten-Reader eingesetzten Mikroplatte ausgesendet wird, bzw. das von mit Licht durchstrahlten Proben in Wells einer in diesen Mikroplatten-Reader eingesetzten Mikroplatte beeinflusst wird. Die Aufnahmeeinrichtung dient zur Aufnahme von zumindest einer Mikroplatte und zum Positionieren der Proben enthaltenden Wells dieser Mikroplatte(n) gegenüber der zumindest einen Messeinrichtung. Die Erfindung betrifft zudem ein entsprechendes Verfahren und spezielle Verwendungen solcher Mikroplatten-Reader.

Aus dem Stand der Technik seit vielen Jahren bekannte, gattungsgemässe Mikroplatten-Reader beruhen auf dem Prinzip des Messens der Lumineszenz und/oder Fluoreszenz von mit einem Reagens versetzten Proben. Mit Lumineszenz oder Fluoreszenz wird allgemein das Aussenden von Licht bezeichnet, das von einer Probe stammt, wobei die Lumineszenz auf das Ablaufen einer chemischen Reaktion in einer Probe und die Fluoreszenz auf das Einstrahlen eines Anregungslichts zurückzuführen ist. Solche Mikroplatten-Reader dienen somit zum Beobachten von Reaktionen in Proben, denen ein Reagens zugegeben wird, oder zum Nachweisen von bestimmten Probenbestandteilen, die zum Fluoreszieren gebracht werden können.

Es sind entsprechende Mikroplatten-Reader bekannt, die - zum Auslösen einer Lumineszenzreaktion - eine Injektorvorrichtung zum Zugeben eines Reagens zu den Proben in Wells der in diesen Mikroplatten-Reader eingesetzten Mikroplatte(n) umfassen. Ebenfalls bekannt sind Mikroplatten-Reader, die eine Beleuchtungseinrichtung zum Bestrahlen oder Durchstrahlen von Proben in Wells der in diesen Mikroplatten-Reader eingesetzten Mikroplatte(n) umfassen. Mit solchen Mikroplatten-Readern werden die in den Proben angeregte Fluoreszenz oder die durch die Proben verursachte Reduktion der Transparenz (die Absorbance) gemessen.

Es ist eine Aufgabe der vorliegenden Erfindung, einen Mikroplatten-Reader, entsprechende Verfahren zum Messen lebender Zellen in einem Mikroplatten-Reader und Verwendungen eines solchen Mikroplatten-Readers vorzuschlagen, welche das Beobachten von Reaktionen in Proben bzw. den Nachweis von bestimmten Probenbestandteilen unter zumindest annähernd physiologischen Bedingungen ermöglichen.

Diese Aufgabe wird in Bezug auf einen ersten Aspekt mit einem Mikroplatten-Reader gemäss den Merkmalen des unabhängigen Anspruchs 1 gelöst.

Eine Hauptfunktion der Trennplatte und des Innengehäuses ist den beiden im Anspruch 1 genannten alternativen Begrenzungsmitteln gemeinsam, nämlich das Abtrennen des Probenraums vom Geräteraum, derart dass die Proben von den Geräten nur im gewünschten Masse beeinflusst werden.

Im Innenraum des Gehäuses kann der Mikroplatten-Reader so ausgestaltet sein, dass mittels der Trennplatte bzw. des Innengehäuses der Probenraum von dem Geräteraum im Wesentlichen lichtdicht und/oder im Wesentlichen gasdicht abgetrennt ist. Dazu kann die Öffnung eine entsprechende Abdichteinrichtung umfassen. Alternativ oder zusätzlich dazu kann eine sich um die Öffnung herum erstreckende Abdichteinrichtung vorgesehen sein. In den beschriebenen Ausführungsformen kann die Abdichteinrichtung so ausgestaltet sein, dass sie primär im Wesentlichen den Durchtritt von Licht durch die Öffnung beeinflusst, und zwar so, dass das von Proben in Wells einer in den Mikroplatten-Reader eingesetzten Mikroplatte ausgesendete oder beeinflusste Licht durch die Öffnung vom Probenraum in den Geräteraum übertreten kann, während anderes (nicht von den Proben ausgesendetes oder beeinflusstes) Licht möglichst vollständig von einem Durchtritt durch die Öffnung abgehalten wird.

In dem Probenraum kann eine Bewegungseinrichtung zum Mischen und/oder Umwälzen von in dem Probenraum anwesendem und/oder einströmendem Gas angeordnet sein.

Die Bewegungseinrichtung kann mindestens eine der folgenden Einrichtungen umfassen: einen Lüfter, eine Einrichtung mit einer oder mehreren Prallplatten, eine Schütteleinrichtung mit einem oder mehreren Paddeln oder ein strukturiertes Düsensystem. Dabei kann die Einrichtung mit der einen oder den mehreren Prallplatten in der Nähe eines Gaseinlasses in den Probenraum angeordnet sein. Die Schütteleinrichtung mit dem einen oder den mehreren Paddeln kann in der Nähe der Aufnahmeeinrichtung angeordnet oder die Aufnahmeeinrichtung selbst sein. Das strukturierte Düsensystem kann eine Vielzahl von Einlassdüsen umfassen, die im Probenraum im Wesentlichen gleichmässig verteilt angeordnet sind, um zu bewirken, dass beim Einlassen von Gas durch die Einlassdüsen die Gasatmosphäre im Probenraum im Wesentlichen gleichförmig bewegt und durchmischt wird. Die Einlassdüsen können entlang einer Gaseinlassleitung, vorzugsweise im Wesentlichen gleich voneinander beabstandet, angeordnet sein. Die Gaseinlassleitung kann sich im Wesentlichen als Ganzes entlang bzw. im Wesentlichen parallel zu einer Wandfläche, etwa einer Decken- oder Bodenfläche, des Probenraums schlängeln. Eine Gesamtanordnung der Einlassdüsen kann eine im Wesentlichen S-förmige Anordnung, eine Mehrzahl von S-förmigen Anordnungen, eine mäanderförmige Anordnung und/oder eine im Wesentlichen spiralförmige Anordnung umfassen. Alternativ oder zusätzlich dazu kann die Gaseinlassleitung sich zumindest abschnittsweise, in einem oder mehreren Abschnitten, in zwei oder mehrere Gaseinlassteilleitungen aufteilen oder aufgabeln.

Die Kontrolleinheit kann einen Rechner mit entsprechender, z.B. darin gespeicherter, Software umfassen, wobei der Rechner mit einem zentralen Rechner des Mikroplatten-Readers verbindbar oder in diesen integriert sein kann. Alternativ dazu kann die Kontrolleinheit einen Rechner mit entsprechender Software umfassen, wobei der Rechner mit einem zentralen Rechner des Mikroplatten-Readers verbindbar und in einem separaten Gehäuse angeordnet sein kann.

Die Kontrolleinheit kann dazu ausgebildet sein, die Zusammensetzung der Gasatmosphäre mit bis zu vier, fünf, sechs oder mehr unterschiedlichen Gasen zu steuern. Dazu umfasst die Kontrolleinheit Gassensoren zum Messen von unterschiedlichen Gasen in dem Probenraum, d.h. zur Steuerung der Zusammensetzung der Gasatmosphäre um die Proben enthaltenden Wells von einer in diesem Mikroplatten-Reader eingesetzten Mikroplatte. Unabhängig davon kann der Mikroplatten-Reader einen von der Kontrolleinheit ansteuerbaren Gaseinlass, insbesondere einen Gaseinlass ohne Gassensor, zum Einlassen von Gas in den Probenraum umfassen.

Mindestens ein Gas der unterschiedlichen Gase kann ausgewählt sein aus einer Gruppe, die Stickstoff (N₂), Kohlendioxid (CO₂), Sauerstoff (O₂), Kohlenmonoxid (CO), Schwefelwasserstoff (H₂S) und Schwefeldioxid (SO₂) umfasst.

Die Kontrolleinheit umfasst bevorzugt einen O₂-Sensor zur Messung und Steuerung des Sauerstoffgehalts der Gasatmosphäre um die Proben enthaltenden Wells von einer in diesem Mikroplatten-Reader eingesetzten Mikroplatte und einen CO₂-Sensor zur Messung und Steuerung des Kohlendioxidgehalts der Gasatmosphäre um die den Proben enthaltenden Wells von in diesen Mikroplatten-Reader eingesetzten Mikroplatte.

Unabhängig davon bzw. zusätzlich dazu kann die Kontrolleinheit dazu ausgebildet ist, die Feuchtigkeit und die Temperatur der Gasatmosphäre zu steuern. Dazu kann die Kontrolleinheit im Probenraum einen Feuchtigkeitssensor zum Messen der Feuchtigkeit der Gasatmosphäre und einen Temperatursensor zum Messen der Temperatur der Gasatmosphäre umfassen.

Unabhängig davon bzw. zusätzlich dazu kann die Kontrolleinheit eine Kühleinrichtung zum Kühlen und/oder eine Heizeinrichtung zum Heizen des Probenraums umfassen. Vorzugsweise ist dabei die Heizeinrichtung an der Trennplatte bzw. an dem Innengehäuse montiert und mit der Gasatmosphäre in dem Probenraum in Wärmetauschkommunikation und die Kühleinrichtung am Boden des Probenraums montiert und mit der Gasatmosphäre in dem Probenraum in Wärmetauschkommunikation. Die Heizeinrichtung kann an einer Platte, die an der Probenraumseite der Trennplatte bzw. an der Innenraumseite des Innengehäuses angeordnet ist, montiert sein. Vorzugsweise ist die Platte mit der Trennplatte bzw. mit dem Innengehäuse im Wesentlichen nicht wärmeleitend verbunden. Durch diese Anordnungen der Heizeinrichtung und der Kühleinrichtung wird einer Kondensation von Feuchtigkeit bzw. Wasser auf der Mikroplatte bzw. an, auf oder in den Wells der Mikroplatte entgegen gewirkt. Die Heizeinrichtung bzw. die Kühleinrichtung kann zum Heizen bzw. Kühlen der Gasatmosphäre in dem Probenraum von der Kontrolleinheit aktiv ansteuerbar sein. Das hier bezüglich der Kühleinrichtung bzw. der Heizeinrichtung Gesagte gilt gleichermassen für die Ausgestaltungen des Mikroplatten-Readers mit einer Trennplatte oder mit einem Innengehäuse.

In dem Mikroplatten-Reader kann eine gesonderte Ventilationseinrichtung, die in dem Geräteraum angeordnet ist, vorgesehen sein. Die Ventilationseinrichtung dient zum Kühlen von im Geräteraum angeordneten Lichtquellen (z.B. Lampen) bzw. anderen Wärme erzeugenden Geräten. Die Ventilationseinrichtung kann unabhängig von der im Probenraum angeordneten Heizeinrichtung bzw. der im Probenraum angeordneten Kühleinrichtung angesteuert bzw. betrieben werden.

In dem Mikroplatten-Reader kann die zweite Messeinrichtung und/oder die dritte Messeinrichtung jeweils einen Lichtleiter, der eine Eintrittsstelle für Licht und eine Austrittsstelle für Licht aufweist, und eine Lichtdetektoreinrichtung, die zum Messen bzw. Detektieren von aus der Austrittsstelle austretendem Licht angeordnet ist, umfassen. Die erste Eintrittsstelle von Licht in die zweite Eintrittsstelle von Licht in die zweite Messeinrichtung und die dritte Eintrittsstelle von Licht in die dritte Messeinrichtung können jeweils im Probenraum angeordnet sein.

Unter dem Begriff Lichtleiter ist hierin eine optische Faser, ein optisches Faserbündel oder ein Spiegelsystem zu verstehen. Sowohl ein als optische Faser, optisches Faserbündel als auch ein als Spiegelsystem ausgebildeter Lichtleiter umfasst eine Eintrittsstelle für Licht, eine Austrittsstelle für Licht und eine sich von der Eintrittsstelle zur Austrittsstelle erstreckende Lichtleitstrecke, entlang der Licht von dem Lichtleiter geleitet werden kann.

In dem Mikroplatten-Reader kann ein jeweiliger Lichtleiter der zweiten und/oder dritten Messeinrichtung als eine optische Faser oder als ein optisches Faserbündel ausgebildet sein. Alternativ dazu kann ein jeweiliger Lichtleiter als ein Spiegelsystem ausgebildet sein. Kombinationen von Licht leitenden Fasern und Spiegeln sind sowohl für die Beleuchtung der Proben als auch für die Detektion des von den Proben stammenden Lichts denkbar.

Die Lichtdetektoreinrichtung einer jeweiligen ersten, zweiten und/oder dritten Messeinrichtung kann im Probenraum oder im Geräteraum angeordnet sein.

In dem Mikroplatten-Reader können die erste Lichtdetektoreinrichtung der ersten Messeinrichtung und/oder die dritte Lichtdetektoreinrichtung der dritten Messeinrichtung im Probenraum angeordnet sein. In dem Mikroplatten-Reader kann die zweite Lichtdetektoreinrichtung der zum Messen der Fluoreszenz in Wells ausgelegten zweiten Messeinrichtung im Geräteraum angeordnet sein.

Der Mikroplatten-Reader umfasst mindestens eine erste Beleuchtungseinrichtung , die zum Durchstrahlen bzw. zum Anstrahlen von Proben in Wells einer in dem Mikroplatten-Reader eingesetzten Mikroplatte mit Licht ausgelegt ist. Die erste Beleuchtungseinrichtung umfasst eine Blitzlampe, eine erste faseroptische Leitung und einen ersten, Wellenlängen selektierenden Monochromator.

Für eine jeweilige der ersten, zweiten bzw. dritten Messeinrichtung kann eine erste, zweite bzw. dritte Beleuchtungseinrichtung bereitgestellt sein. Es ist jedoch auch möglich, vorzusehen, dass eine Beleuchtungseinrichtung gemeinsam für die erste und zweite Messeinrichtung, für die zweite und dritte Messeinrichtung, für die erste und dritte Messeinrichtung bzw. gemeinsam für die erste, zweite und dritte Messeinrichtung bereitgestellt ist.

Eine jeweilige Lichtquelle einer Beleuchtungseinrichtung kann ausgewählt sein aus einer Gruppe, die einen Laser, eine Blitzlampe, eine LED (Licht emittierende Diode) und eine Laserdiode umfasst, wobei die erste Beleuchtungseinrichtung eine Blitzlampe umfasst.

In bestimmten Verwendungen des Mikroplatten-Readers kann es wichtig sein, eine Kinetik, insbesondere eine Kinetik des Wachstums bzw. der Veränderung, von Zellkulturen, die als Proben in einem Well einer Mikroplatte angeordnet sind, zu messen bzw. erfassen. Das Messen bzw. Erfassen der Kinetik kann durch Messen bzw. Erfassen, insbesondere einer Veränderung als Funktion der Zeit, der Absorbance, der Fluoreszenz und/oder der Lumineszenz der Zellkultur (Probe), vorzugsweise der Lumineszenz der Zellkultur (Probe), bewerkstelligt werden.

Zum Messen bzw. Erfassen von zeitlich relativ schnell ablaufenden Kinetiken kann es erforderlich, insbesondere vorteilhaft sein, dass die Lichtquelle der Beleuchtungseinrichtung zeitlich kurze Lichtpulse erzeugt. Daher kann ein gepulster Laser, eine Blitzlampe, eine in einem Pulsbetrieb betriebene LED (Licht emittierende Diode) bzw. eine in einem Pulsbetrieb betriebene Laserdiode eine dafür geeignete Lichtquelle sein, wobei die erste Beleuchtungseinrichtung eine Blitzlampe umfasst.

Als Lichtquelle kann in dem Mikroplatten-Reader eine nicht aktiv gekühlte Blitzlampe vorgesehen sein. Diese Blitzlampe kann im Geräteraum angeordnet sein. Ferner kann von dieser Blitzlampe erzeugtes Licht mittels eines Lichtleiters zu einer Probe, die in einem Well von einer in den Mikroplatten-Reader eingesetzten Mikroplatten enthalten ist, zum Anstrahlen und/oder Durchstrahlen der Probe geleitet werden im Rahmen des Schutzumfangs des Anspruchs 1.

Der Mikroplatten-Reader kann eine erste Beleuchtungseinrichtung umfassen, die zum Durchstrahlen von Proben in Wells einer in den Mikroplatten-Reader eingesetzten Mikroplatte mit Licht ausgelegt ist und die im Geräteraum angeordnet ist. Dabei kann die erste Messeinrichtung im Probenraum angeordnet sein. Die zweite Messeinrichtung kann im Geräteraum angeordnet sein. Die dritte Messeinrichtung kann im Probenraum oder im Geräteraum angeordnet sein. Dabei können die erste Eintrittsstelle von Licht in die erste Messeinrichtung, die zweite Eintrittsstelle von Licht in die zweite Messeinrichtung und die dritte Eintrittsstelle von Licht in die dritte Messeinrichtung im Probenraum angeordnet sein.

Der Mikroplatten-Reader kann eine erste Beleuchtungseinrichtung umfassen, die zum Durchstrahlen von Proben in Wells einer in den Mikroplatten-Reader eingesetzten Mikroplatte mit Licht ausgelegt ist. Dabei kann eine Austrittsstelle der ersten Beleuchtungseinrichtung im Probenraum angeordnet sein. Der Mikroplatten-Reader kann ferner eine zweite Beleuchtungseinrichtung umfassen, die im Geräteraum angeordnet ist und die zum Bestrahlen von Proben in Wells einer in den Mikroplatten-Reader eingesetzten Mikroplatte mit Licht ausgelegt ist. Die zweite Beleuchtungseinrichtung kann ausgewählt sein aus der Gruppe, die einen Laser, eine Blitzlampe, eine LED (Licht emittierende Diode) und eine Laserdiode umfasst.

Die zweite bzw. dritte Messeinrichtung kann eine zweite bzw. dritte Optik umfassen, wobei zumindest eine dieser Optiken und/oder eine eingesetzte Mikroplatte in Richtung einer Z-Achse eines kartesischen Koordinatensystems relativ zueinander bewegbar ausgebildet sind. Dabei kann die eingesetzte Mikroplatte in Richtung der Z-Achse bewegbar ausgebildet sein. Alternativ bzw. zusätzlich dazu kann die erste, zweite bzw. dritte Optik in Richtung der Z-Achse bewegbar ausgebildet sein. Vorzugsweise ist die erste, zweite bzw. dritte Optik durch die mindestens eine Öffnung in der Trennplatte bzw. einer Wand, etwa einer Deckenwand, des Innengehäuses teilweise in den Probenraum absenkbar ausgebildet.

In dem entsprechenden Verfahren zum Messen lebender Zellen in einem Mikroplatten-Reader (siehe unten) bzw. bei der Verwendung eines Mikroplatten-Readers (siehe unten) kann es vorkommen, dass ein Reagens zu mindestens einer Probe in mindestens einem Well der Mikroplatte - etwa mittels einer Injektorvorrichtung - zugegeben wird und damit eine chemische Reaktion ausgelöst wird. Mit dem Auslösen der chemischen Reaktion kann eine Lumineszenzreaktion bzw. eine Veränderung des Lumineszenzverhaltens, eine Veränderung der Fluoreszenz und/oder eine Veränderung der Absorbance der Probe einhergehen. Diese Veränderungen können, etwa mittels zumindest einer der Messeinrichtungen, z.B. durch Beobachten (Messen bzw. Erfassen) der Lumineszenz und/oder der Fluoreszenz und/oder der Absorbance der Probe, detektiert werden.

Zum Zugeben eines Reagens kann der Mikroplatten-Reader eine entsprechende Injektorvorrichtung umfassen, die zum Zugeben eines Reagens zu den Proben in Wells einer in den Mikroplatten-Reader eingesetzten Mikroplatte ausgebildet ist. Die Injektorvorrichtung kann beispielsweise Reagenzien abgeben, die in einem Well, das sich gerade in einer optischen Achse der dritten Messeinrichtung befindet, eine chemische Reaktion z.B. eine damit einhergehende Lumineszenzreaktion auslösen können. Die Beobachtung (das Messen bzw. Erfassen) einer chemischen Reaktion, z.B. durch Beobachten einer Lumineszenzreaktion, kann in einem gleichen Well im Wesentlichen simultan zu der Zugabe eines Reagens und ggf. in einem sich daran anschliessenden Zeitintervall erfolgen. Alternativ bzw. zusätzlich dazu kann die Beobachtung (das Messen bzw. Erfassen) einer chemischen Reaktion, z.B. durch Beobachten einer Lumineszenzreaktion, auch in einem anderen Well als demjenigen Well, in den das Reagens aktuell zugegeben wird, erfolgen. Dies kann insbesondere im Wesentlichen simultan zu der Zugabe des Reagens in einen Well und ggf. in einem sich daran anschliessenden Zeitintervall erfolgen. Bevorzugt ist es, ein Reagens zu einer Probe in einem Well zuzugeben und simultan dazu in einem dazu beabstandeten, z.B. benachbarten, Well eine chemische Reaktion zu beobachten. In diesem Sinne kann nacheinander im Wesentlichen in jedem Well auf der Mikroplatte ein Reagens zu einer Probe zugegeben werden und in einem beabstandeten, z.B. benachbarten, Well simultan eine chemische Reaktion etwa durch Beobachten (Messen bzw. Erfassen) der Lumineszenz und/oder der Fluoreszenz und/oder der Absorbance der in dem beabstandeten, z.B. benachbarten, Well enthaltenen Probe beobachtet werden. Mit dieser Vorgehensweise kann Well für Well der Mikroplatte "stimuliert" (d.h. der darin enthaltenen Probe ein Reagens zugegeben) werden und um eine Zeitdauer versetzt die durch die Stimulation ausgelöste chemische Reaktion beobachtet (gemessen bzw. erfasst) werden. Dabei ist die Zeitdauer des Zeitversatzes bestimmt durch den geometrischen Versatz (d.h. lateralen Abstand in einer X-Y-Ebene) zwischen den jeweiligen beabstandeten, z.B. benachbarten, Wells und der Geschwindigkeit, mit der die Mikroplatte und die zum Beobachten der chemischen Reaktion verwendete Messeinrichtung (genauer: deren optische Achse), insbesondere in einer Ebene parallel zur Mikroplatte, relativ zueinander bewegt werden.

Diese Aufgabe wird in Bezug auf einen zweiten Aspekt durch ein Verfahren mit den Merkmalen des unabhängigen Anspruchs 15 gelöst.

In diesem Verfahren kann die Reihenfolge von Arbeitsschritten, insbesondere der Schritte b) bis e), vertauscht werden. So kann beispielsweise der Schritt d) vor dem Schritt c) oder vor dem Schritt b) ausgeführt und/oder z.B. der Schritt e) vor dem Schritt c) oder vor dem Schritt d) ausgeführt werden.

Das Verfahren kann ferner zumindest einen oder mehrere der folgenden Schritte umfassen:
f) Messen von Licht, das von mit Licht durchstrahlten Proben in Wells einer in den Mikroplatten-Reader eingesetzten Mikroplatte beeinflusst wird, zum Messen der Absorbance der Probe,
g) Messen von Licht, das von mit Licht angestrahlten Proben in Wells einer in den Mikroplatten-Reader eingesetzten Mikroplatte ausgesendet wird, zum Messen der Fluoreszenz der Probe, und
h) Messen von Licht, das von Proben in Wells einer in den Mikroplatten-Reader eingesetzten Mikroplatte ausgesendet wird, zum Messen der Lumineszenz der Probe.

In dem Probenraum kann eine definierte Gasatmosphäre eingestellt werden und mit der eingestellten Gasatmosphäre des oben genannten Schrittes d) kann zumindest einer oder mehrere der vorgenannten Schritte f), g) und h) ausgeführt werden.

In dem Verfahren kann in den Probenraum einströmendes und/oder dort anwesendes Gas gemischt und/oder umgewälzt werden mittels einer in dem Probenraum angeordneten Bewegungseinrichtung.

In dem Verfahren kann die Zusammensetzung der Gasatmosphäre im Probenraum gesteuert werden mittels einer Kontrolleinheit. Dabei kann Gas in den Probenraum durch einen Gaseinlass eingelassen werden, wobei der Gaseinlass von der Kontrolleinheit angesteuert wird. In dem Verfahren kann der Anteil in der Gasatmosphäre im Probenraum von mindestens einem bestimmten Gas aus unterschiedlichen Gasen gesteuert werden, wobei das bestimmte Gas ausgewählt ist aus einer Gruppe, die Stickstoff (N₂), Kohlendioxid (CO₂), Sauerstoff (O₂), Kohlenmonoxid (CO), Schwefelwasserstoff (H₂S) und/oder Schwefeldioxid (SO₂) umfasst. In dem Verfahren können die Feuchtigkeit und/oder die Temperatur der Gasatmosphäre in dem Probenraum mittels der Kontrolleinheit gesteuert werden. In dem Verfahren kann in dem Geräteraum die Luft mittels einer Ventilationseinrichtung ventiliert werden.

In dem Verfahren zum Messen lebender Zellen bzw. bei der Verwendung eines Mikroplatten-Readers (siehe unten) kann ein Reagens zu mindestens einer Probe in mindestens einem Well der Mikroplatte mittels einer Injektorvorrichtung zugegeben werden. Dadurch kann eine mittels zumindest einer der Messeinrichtungen detektierbare chemische Reaktion bewirkt werden. Dabei kann insbesondere auch eine mit der chemischen Reaktion einhergehende Lumineszenzreaktion bzw. eine Veränderung des Lumineszenzverhaltens, eine Veränderung der Fluoreszenz und/oder eine Veränderung der Absorbance der Probe ausgelöst werden. Beispielsweise kann ein Reagens in einem Well, der sich gerade in einer optischen Achse der dritten Messeinrichtung befindet, abgegeben werden und dort eine chemische Reaktion auslösen.

Die Beobachtung (das Messen bzw. Erfassen) einer chemischen Reaktion, z.B. durch Beobachten der ausgelösten Lumineszenzreaktion, kann in einem gleichen Well im Wesentlichen simultan zu der Zugabe eines Reagens und ggf. in einem sich daran anschliessenden Zeitintervall erfolgen. Alternativ bzw. zusätzlich dazu kann die Beobachtung (das Messen bzw. Erfassen) einer chemischen Reaktion, z.B. durch Beobachten der ausgelösten Lumineszenzreaktion, auch in einem anderen Well als demjenigen Well, in dem das Reagens aktuell zugegeben wird, erfolgen. Dies kann insbesondere im Wesentlichen simultan zu der Zugabe des Reagens in einen Well und ggf. in einem sich daran anschliessenden Zeitintervall erfolgen.

Wie bereits gesagt ist es bevorzugt, ein Reagens zu einer Probe in einem Well zuzugeben und simultan dazu in einem dazu beabstandeten, z.B. benachbarten, Well eine chemische Reaktion zu beobachten.

Eine durch Zugabe eines Reagens ausgelöste chemische Reaktion in einer Probe (etwa einer Zellkultur) bzw. eine Zellkultur kann durch Beobachten (Messen bzw. Erfassen) einer Kinetik, insbesondere einer Kinetik des Wachstums bzw. der Veränderung der Probe bzw. der Zellkultur, die in einem Well einer Mikroplatte angeordnet ist, beobachtet bzw. identifiziert werden. In dem Verfahren können Proben in Wells einer in den Mikroplatten-Reader eingesetzten Mikroplatte mit Licht durchstrahlt bzw. Proben in den Wells mit Licht angestrahlt werden mittels einer Beleuchtungseinrichtung.

Proben in Wells der eingesetzten Mikroplatte können auch mittels einer zweiten Beleuchtungseinrichtung mit Licht angestrahlt werden. Die zweite Beleuchtungseinrichtung kann im Geräteraum angeordnet werden. Sie kann vorzugsweise ausgewählt werden aus einer Gruppe, die einen Laser, eine Blitzlampe, eine LED (Licht emittierende Diode) und eine Laserdiode umfasst. In dem Verfahren kann die Absorbance einer Probe, und/oder die Fluoreszenz der Probe und/oder die Lumineszenz der Probe, jeweils mittels einer im Probenraum oder im Geräteraum angeordneten Messeinrichtung, gemessen werden. Insbesondere können die Absorbance der Probe mittels einer im Probenraum angeordneten, ersten Messeinrichtung bzw. die Fluoreszenz der Probe mittels einer im Geräteraum angeordneten, zweiten Messeinrichtung gemessen werden. Auch kann die Lumineszenz der Probe mittels einer im Probenraum oder im Geräteraum angeordneten, dritten Messeinrichtung gemessen werden.

In dem Mikroplatten-Reader, in dem das Verfahren ausgeführt kann, kann die Kontrolleinheit einen O₂-Sensor und/oder einen CO₂-Sensor zur Steuerung des Sauerstoff- und/oder Kohlendioxidgehalts der Gasatmosphäre um die die Proben enthaltenden Wells einer in diesen Mikroplatten-Reader eingesetzten Mikroplatte umfassen. Dabei kann die CO₂- bzw. O₂-Konzentration dieser Gasatmosphäre beim Vermessen mikroaerophiler, fakultativ anaerober oder obligat anaerober Mikroorganismen, Pilzen oder eukaryotischer Zellen durch gezieltes Einlassen von Kohlendioxid und/oder Stickstoff auf einem definierten Wert gehalten werden.

In dem oben beschriebenen Verfahren kann:
d1) beim Steuern der Zusammensetzung der Gasatmosphäre in dem Probenraum die Konzentration eines in der Gasatmosphäre bereits vorhandenen Gases, wie etwa Sauerstoff (O₂) oder Kohlendioxid (CO₂), erniedrigt werden, indem ein chemisch inaktives Gas, wie etwa Stickstoff oder ein Edelgas in den Probenraum eingelassen wird (wodurch die Gasatmosphäre einschliesslich der Konzentration des bereits vorhandenen Gases "verdünnt" bzw. teilweise verdrängt wird) und die Konzentration dieses vorhandenen Gases mittels eines auf dieses vorhandene Gas ansprechenden Sensors, etwa eines O₂-Sensors oder eines CO₂-Sensors, gemessen wird, oder es kann
d2) beim Steuern der Zusammensetzung der Gasatmosphäre in dem Probenraum die Konzentration eines in der Gasatmosphäre bereits vorhandenen Gases, wie etwa Sauerstoff (O₂) oder Kohlendioxid (CO₂), erhöht werden, indem mehr von diesem Gas in den Probenraum eingelassen wird (wodurch insbesondere die Konzentration dieses Gases gezielt erhöht wird) und die Konzentration dieses Gases mittels eines auf dieses Gas ansprechenden Sensors gemessen wird, oder es kann
d3) beim Steuern der Zusammensetzung der Gasatmosphäre in dem Probenraum die Konzentration eines in der Gasatmosphäre bereits oder noch nicht vorhandenen Gases, wie etwa Schwefelwasserstoff (H₂S) oder Kohlenmonoxid (CO), erhöht werden, indem mehr von diesem Gas in den Probenraum eingelassen wird (wodurch insbesondere die Konzentration dieses Gases gezielt erhöht wird) und die Konzentration dieses Gases mittels eines auf dieses Gas ansprechenden Sensors gemessen werden.

Die oben genannte Aufgabe wird in Bezug auf einen dritten Aspekt mit der erfindungsgemässen Verwendung des oben eingeführten, erfindungsgemässen Mikroplatten-Readers bzw. des oben eingeführten, erfindungsgemässen Verfahrens gelöst. Diese Verwendungen betreffen das Messen lebender Zellen in einem Mikroplatten-Reader, wobei die lebenden Zellen ausgewählt sind aus einer Gruppe, die mikroaerophile, fakultativ anaerobe und obligat anaerobe Mikroorganismen, sowie Pilze und eukaryotische Zellen umfasst.

Zusätzliche erfinderische Merkmale ergeben sich jeweils aus den abhängigen Ansprüchen.

Besonders bevorzugt ist die Verwendung eines derartigen Mikroplatten-Readers beim Vermessen mikroaerophiler oder fakultativ anaerober Mikroorganismen bei einer definierten O₂-Konzentration. Auch bevorzugt sind Verfahren zum Messen lebender Zellen in einem Mikroplatten-Reader, wobei die lebenden Zellen ausgewählt sind aus einer Gruppe, die mikroaerophile, fakultativ anaerobe und obligat anaerobe Mikroorganismen sowie Pilze und eukaryotische Zellen umfasst. Als Mikroplatten werden Multiwellplatten bzw. Mikrotiterplatten nach dem ANSI-SBS Standard 2004 bezeichnet, die z.B. 6, 12, 24, 48, 96, 384 oder 1536 Wells aufweisen können.

Der erfindungsgemässe Mikroplatten-Reader umfasst die folgenden Vorteile:
- Die Mikroplatten mit den zu vermessenden Proben müssen nicht dauernd zwischen einem Inkubator und dem Messgerät hin und her transferiert werden. Damit fallen derartige Transfers weg, während denen die Zellen bzw. Zellkulturen der Umgebungsluft ausgesetzt werden und durch diese Umgebungsluft so beeinflusst werden können, dass verfälschte Messergebnisse resultieren.
- Die Messungen in Bezug auf Lumineszenz und/oder Fluoreszenz und/oder Absorbance können vollständig automatisiert erfolgen, so dass nach dem Beschicken des erfindungsgemässen Mikroplatten-Readers kein Operator anwesend sein muss.

- Im Vergleich zu herkömmlichen (beispielsweise CO₂-) Inkubatoren ohne integrierte Fluoreszenzmessung können mit dem erfindungsgemässen Mikroplatten-Reader Langzeit-Messungen durchgeführt werden, so dass es zu keinen sogenannten "Nachtfenstern" kommen kann, während welchen keine Daten erfasst werden können.
- Das Steuern der O₂- und/oder CO₂-Konzentration in der Atmosphäre über den bzw. in der Umgebung der Wells von Mikroplatten ermöglicht das Vermessen mikroaerophiler, fakultativ anaerober oder obligat anaerober Mikroorganismen, Pilze oder eukaryotischer Zellen bei einer definierten O₂-und/oder CO₂-Konzentration.

Der erfindungsgemässe Mikroplatten-Reader und dessen erfindungsgemässe Verwendung werden nun an Hand von schematischen Abbildungen näher erläutert, welche beispielhafte und bevorzugte Ausführungsformen zeigen, ohne den Umfang der vorliegenden Erfindung einzuschränken. Dabei zeigt:
- Fig. 1: einen stark schematisierten Vertikalschnitt durch einen erfindungsgemässen Mikroplatten-Reader gemäss einer bevorzugten ersten Ausführungsform mit einem Gehäuse-Innenraum, der mittels einer Trennplatte in einen Geräteraum und in einen Probenraum unterteilt ist;
- Fig. 2: einen stark schematisierten Vertikalschnitt durch einen erfindungsgemässen Mikroplatten-Reader gemäss einer bevorzugten zweiten Ausführungsform mit einem Gehäuse-Innenraum, der mittels einer Trennplatte in einen Geräteraum und in einen Probenraum unterteilt ist;
- Fig. 3: einen stark schematisierten Vertikalschnitt durch einen erfindungsgemässen Mikroplatten-Reader gemäss einer bevorzugten dritten Ausführungsform mit einem Gehäuse-Innenraum, der mittels einer Trennplatte in einen Geräteraum und in einen Probenraum unterteilt ist;
- Fig. 4: eine beispielhafte Anordnung eines O₂-Sensors, eines CO₂-Sensors, eines Lüfters und eines Gaseinlasses an der Innenseite der Rückwand eines erfindungsgemässen Mikroplatten-Readers;
- Fig. 5: unter Verwendung eines erfindungsgemässen Mikroplatten-Readers mit oder ohne CO₂-Regelung erstellte Wachstumskurven von eukaryotischen Tumorzellen in einem Medium mit Serum (MMS);
- Fig. 6: unter Verwendung eines erfindungsgemässen Mikroplatten-Readers mit oder ohne CO₂-Regelung erstellte Wachstumskurven von eukaryotischen Tumorzellen in einem Medium ohne Serum (MOS).

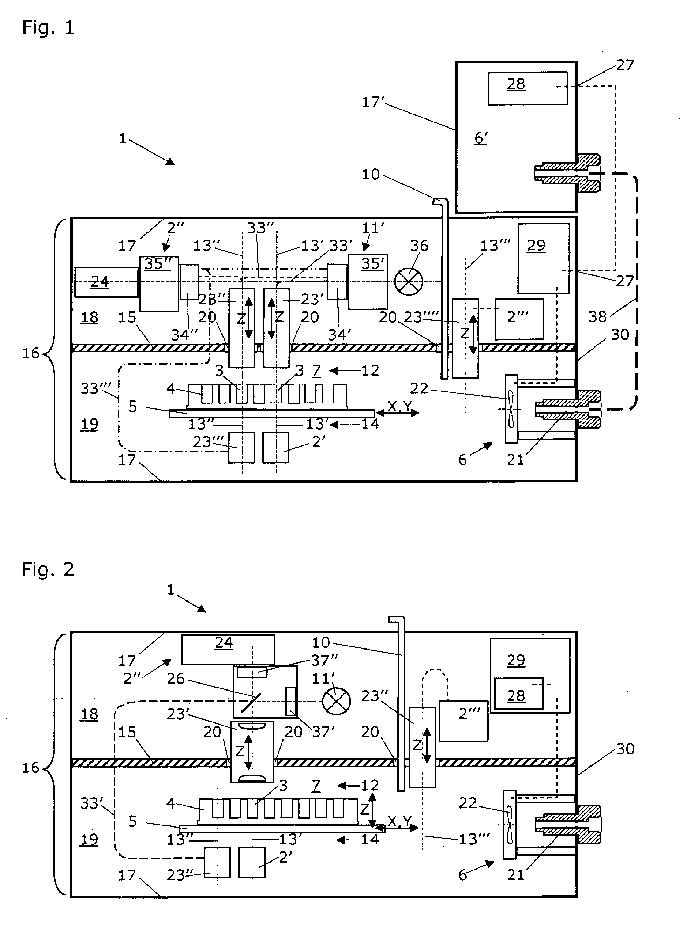

Figur 1 zeigt einen stark schematisierten Vertikalschnitt durch einen erfindungsgemässen Mikroplatten-Reader 1 gemäss einer bevorzugten ersten Ausführungsform mit einem Gehäuse 17, dessen Innenraum 16 mittels einer Trennplatte 15 in einen Geräteraum 18 und in einen Probenraum 19 unterteilt ist. Dieser Mikroplatten-Reader 1 umfasst zumindest eine Messeinrichtung 2',2",2"' zum Detektieren von Licht.

In der Fig. 1 ist gemäss einer bevorzugten ersten Variante des erfindungsgemässen Mikroplatten-Readers 1 eine erste Messeinrichtung 2' dargestellt, mit welcher Licht, das von mit Licht durchstrahlten Proben in Wells 3 einer in diesen Mikroplatten-Reader 1 eingesetzten Mikroplatte 4 beeinflusst wird (also die Absorbance der Probe), gemessen werden kann. Diese erste Messeinrichtung 2' ist vorzugsweise im Probenraum 19 des Mikroplatten-Readers 1 angeordnet und eine erste Beleuchtungseinrichtung 11' zum Durchstrahlen der Proben in den Wells 3 einer Mikroplatte 4 ist bevorzugt im Geräteraum 18 des Mikroplatten-Readers 1 angeordnet. Dadurch ist die erste Messeinrichtung 2' zum Detektieren von Licht, das von Proben in Wells 3 einer in diesen Mikroplatten-Reader 1 eingesetzten Mikroplatte 4 beeinflusst wird, auf einer zweiten Seite 14 (also auf der Unterseite) einer in diesen Mikroplatten-Reader 1 eingesetzten Mikroplatte 4 und in Richtung einer ersten optischen Achse 13' angeordnet. Folglich ist die erste Beleuchtungseinrichtung 11' zum Durchstrahlen der Proben auf einer ersten Seite 12 (also auf der Oberseite) einer in diesen Mikroplatten-Reader 1 eingesetzten Mikroplatte 4 und ebenfalls in Richtung dieser ersten optischen Achse 13' angeordnet. Die Zuordnung der ersten Beleuchtungseinrichtung 11' zur ersten optischen Achse 13' erfolgt hier mit einer ersten faseroptischen Leitung 33'. Zu der ersten Beleuchtungseinrichtung 11' gehören ein erster Faserschieber 34', ein erster, Wellenlängen selektierender Monochromator 35' und eine Blitzlampe 36. Somit wird hier das Licht der Blitzlampe 36 zum Durchleuchten der Proben mittels der ersten faseroptischen Leitung 33' in die Richtung der ersten optischen Achse 13' und via eine erste Optik 23' auf die Proben gelenkt. Diese erste Optik 23' kann in Richtung einer Z-Achse eines kartesischen Koordinatensystems höhenverschiebbar ausgebildet sein (vgl. Doppelpfeil Z). In diesem Fall ist zwischen der ersten Beleuchtungseinrichtung 11' und der ersten Messeinrichtung 2', jedoch bevorzugt im Probenraum 19, eine Aufnahmeeinrichtung 5 zur Aufnahme von zumindest einer Mikroplatte 4 und zum Positionieren der Proben enthaltenden Wells 3 dieser Mikroplatte(n) 4 gegenüber der ersten Messeinrichtung 2' und damit auch gegenüber der ersten optischen Achse 13' angeordnet.

In der Fig. 1 ist gemäss einer bevorzugten zweiten Variante des erfindungsgemässen Mikroplatten-Readers 1 eine zweite Messeinrichtung 2" dargestellt, mit welcher Licht, das von Proben in Wells 3 einer in diesen Mikroplatten-Reader 1 eingesetzten Mikroplatte 4 ausgesendet wird (also die Fluoreszenz der Probe), in Bezug auf eine zweite optische Achse 13" gemessen werden kann. Diese zweite Messeinrichtung 2" umfasst eine Fotomultiplier-Röhre (PMT) 24, einen zweiten, Wellenlängen selektierender Monochromator 35" und einen zweiten Faserschieber 34". Zu dieser Fluoreszenz-Messung wird wieder die erste Beleuchtungseinrichtung 11' des Mikroplatten-Readers 1 zum Bestrahlen (Anregen) der Proben in den Wells 3 einer Mikroplatte 4 verwendet. Die Zuordnung der ersten Beleuchtungseinrichtung 11' zur zweiten optischen Achse 13" erfolgt hier mit einer zweiten faseroptischen Leitung 33" und über eine zweite Optik 23". Diese zweite Optik 23" kann in Richtung einer Z-Achse eines kartesischen Koordinatensystems höhenverschiebbar ausgebildet sein (vgl. Doppelpfeil Z). Somit wird hier das Licht der Blitzlampe 36 zum Bestrahlen der Proben mittels der zweiten faseroptischen Leitung 33" in die Richtung der zweiten optischen Achse 13" gelenkt.

Zum Detektieren der von den Proben ausgehenden Fluoreszenz können zwei unterschiedliche Anordnungen verwendet werden:
- Beim sogenannten "Top Reading" wird die zweite Optik 23" oberhalb der Mikroplatte 4 verwendet, welche über die zweite faseroptische Leitung 33" mit dem zweiten Faserschieber 34" verbunden ist, so dass die Fotomultiplier-Röhre (PMT) 24 zum Detektieren der Fluoreszenz jeder einzelnen Probe in einem Well 3 der Mikroplatte 4 verwendet werden kann.
- Beim sogenannten "Bottom Reading" befindet sich eine dritte Optik 23"' unterhalb der Mikroplatte 4 und ist über eine dritte faseroptische Leitung 33"' mit dem ersten und zweiten Faserschieber 34,'34" verbunden, so dass dieselbe Beleuchtungseinrichtung 11' und dieselbe Messeinrichtung 2" zum Anregen und Detektieren der Fluoreszenz jeder einzelnen Probe in einem Well 3 der Mikroplatte 4 verwendet werden können.

In diesem Fall ist die Aufnahmeeinrichtung 5 zur Aufnahme von zumindest einer Mikroplatte 4 und zum Positionieren der Proben enthaltenden Wells 3 dieser Mikroplatte(n) 4 gegenüber der ersten oder zweiten Messeinrichtung 2',2" bevorzugt im Probenraum 19 und unterhalb der vorzugsweise im Geräteraum 18 untergebrachten ersten Beleuchtungseinrichtung 11', angeordnet.

In der Fig. 1 ist gemäss einer bevorzugten dritten Variante des erfindungsgemässen Mikroplatten-Readers 1 eine dritte Messeinrichtung 2''' dargestellt, mit welcher Licht, das von Proben in Wells 3 einer in diesen Mikroplatten-Reader 1 eingesetzten Mikroplatte 4 ausgesendet wird (also die Lumineszenz der Probe), gemessen werden kann. Zu dieser Lumineszenz-Messung wird eine, vorzugsweise im Geräteraum 18 des Mikroplatten-Readers 1 und in Bezug auf eine dritte optische Achse 13"' angeordnete, dritte Messeinrichtung 2'" verwendet. Zum Auslösen einer chemischen Reaktion, mit der eine Lumineszenzreaktion bzw. eine Veränderung des Lumineszenzverhaltens, und/oder eine Veränderung der Fluoreszenz und/oder eine Veränderung der Absorbance in oder an den Proben in den Wells 3 einer Mikroplatte 4 einhergehen kann, wird eine, vorzugsweise in den Probenraum 19 des Mikroplatten-Reader 1 reichende, an sich bekannte, Injektorvorrichtung 10 verwendet. Diese Injektorvorrichtung 10 ist vorzugsweise so gebaut und angeordnet, dass die die chemische Reaktion, z.B. eine Lumineszenzreaktion, auslösenden Reagenzien in ein Well 3 der Mikroplatte 4 zugegeben werden können, welches sich gerade in der dritten optischen Achse 13"' und damit gerade über der dritten Messeinrichtung 2'" befindet.

Eine bevorzugte Ausführungsform des Mikroplatten-Readers 1 ist so ausgestaltet, dass ein Reagens zu einer Probe in einem Well 3 zuzugeben und simultan dazu in einem dazu beabstandeten, z.B. benachbarten, Well eine chemische Reaktion beobachtet wird. Auf diese Weise wird nacheinander im Wesentlichen in jedem Well 3 auf der Mikroplatte 4 ein Reagens zu einer Probe zugegeben und in einem beabstandeten, z.B. benachbarten, Well 3 simultan eine chemische Reaktion beobachtet, etwa durch Messen bzw. Erfassen der Lumineszenz und/oder der Fluoreszenz und/oder der Absorbance der in dem beabstandeten Well enthaltenen Probe. Mit dieser Vorgehensweise wird Well für Well der Mikroplatte 4 "stimuliert" (d.h. der darin enthaltenen Probe ein Reagens zugegeben) und um eine Zeitdauer versetzt wird die durch die Stimulation ausgelöste chemische Reaktion beobachtet (gemessen bzw. erfasst). Dabei ist die Zeitdauer des Zeitversatzes bestimmt durch den geometrischen Versatz (d.h. lateralen Abstand in der X-Y-Ebene) zwischen den jeweils betroffenen (voneinander beabstandeten und z.B. benachbarten) Wells 3 und der Geschwindigkeit, mit der die Mikroplatte 4 und die zum Beobachten der chemischen Reaktion verwendete Messeinrichtung 2', 2", 2"' (bzw. deren optische Achse 13', 13", 13"') parallel zur X-Y-Ebene relativ zueinander bewegt werden.

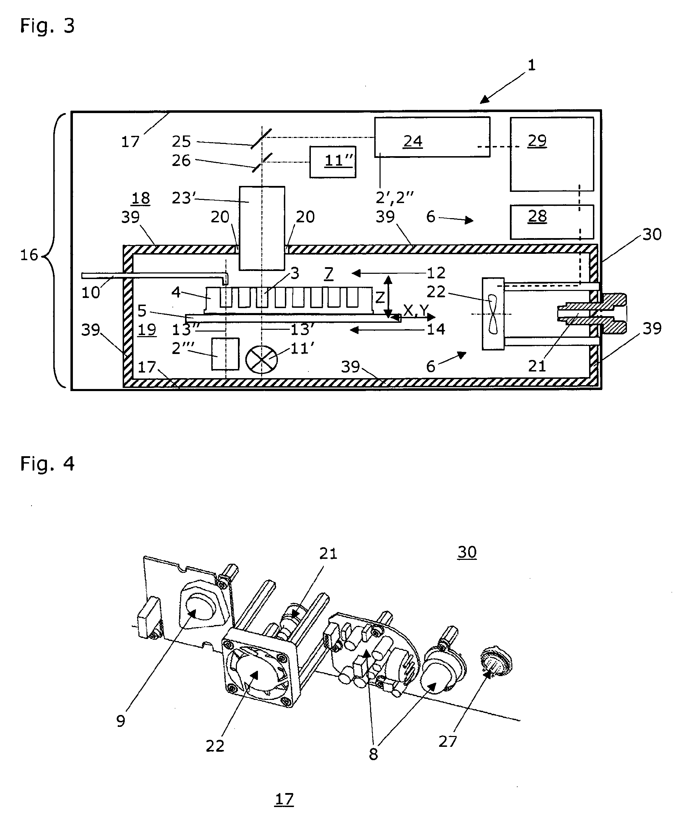

Eine entsprechende Injektorvorrichtung 10 ist auch in den in den Figuren 2 und 3 gezeigten Ausführungsformen eines Mikroplatten-Readers vorgesehen, und das hier bezüglich der Injektorvorrichtung 10 und deren Funktion bzw. Verwendung Gesagte gilt entsprechend auch für die dort gezeigten Ausführungsformen.

Die dritte Messeinrichtung 2''' umfasst vorzugsweise eine vierte Optik 23"", die in Richtung einer Z-Achse eines kartesischen Koordinatensystems bewegbar ausgebildet sein kann (vgl. Doppelpfeil Z). Speziell bevorzugt sind die ersten, zweiten, dritten und vierten Optiken 23', 23", 23"' und 23"" jeweils durch eine Öffnung 20 in der Trennplatte 15 teilweise in den Probenraum 19 absenkbar ausgebildet.

Auf das Gehäuse 17 des Mikroplatten-Readers 1 ist eine separate Kontrolleinheit 6' aufgesetzt, wobei die Anordnung eines O₂-Sensors 8, eines CO₂-Sensors 9, eines Lüfters 22 und eines Gaseinlasses 21 an der Innenseite der Rückwand 30 des Gehäuses 17 des Mikroplatten-Readers 1 vorgesehen sind (vgl. Fig. 4). Die Kontrolleinheit 6 des Mikroplatten-Readers 1 umfasst bevorzugt einen O₂-Sensor 8 zur Messung und Steuerung des Sauerstoffgehalts der Gasatmosphäre 7 im Probenraum 19, d.h. in der Umgebung der Proben enthaltenden Wells 3 von in diesen Mikroplatten-Reader 1 eingesetzten Mikroplatten 4. Besonders bevorzugt umfasst die Kontrolleinheit 6 des Mikroplatten-Readers 1 alternativ oder ergänzend zu diesem O₂-Sensor 8 einen CO₂-Sensor 9 zur Messung und Steuerung des Kohlendioxidgehalts der Gasatmosphäre 7 um die den Proben enthaltenden Wells 3 von in diesen Mikroplatten-Reader 1 eingesetzten Mikroplatte(n) 4. Die Kontrolleinheit 6 ist somit teilweise innerhalb und teilweise ausserhalb des Gehäuses 17 des Mikroplatten-Readers 1 angeordnet und über elektrische Kontakte 27 und Gasleitungen 38 mit dem Mikroplatten-Reader 1 verbunden bzw. verbindbar. Die separate Kontrolleinheit 6' ist direkt an die notwendigen Druckflaschen für die zu verwendenden Gase angeschlossen. Die entsprechenden Ventile und Drosseln für die benötigten Gasanschlüsse sind in die separate Kontrolleinheit 6' eingebaut (nicht gezeigt). Alternativ können auch die Ventile und Drosseln auf den Gasdruckflaschen verwendet werden, wobei diese Ventile vorzugsweise als elektrisch gesteuerte Magnetventile (des Typs stromlos geschlossen) ausgebildet sind. Nicht gezeigt in der Fig. 1 sind unter anderem die Bedienungselemente, Anzeigeelemente und Stromversorgungen für die separate Kontrolleinheit 6' und den Mikroplatten-Reader 1.

Figur 2 zeigt einen stark schematisierten Vertikalschnitt durch einen erfindungsgemässen Mikroplatten-Reader 1 gemäss einer bevorzugten zweiten Ausführungsform mit einem Gehäuse 17, dessen Innenraum 16 mittels einer Trennplatte 15 in einen Geräteraum 18 und in einen Probenraum 19 unterteilt ist. Dieser Mikroplatten-Reader 1 umfasst zumindest eine Messeinrichtung 2',2",2"' zum Detektieren von Licht.

In der Fig. 2 ist gemäss einer bevorzugten ersten Variante des erfindungsgemässen Mikroplatten-Readers 1 eine erste Messeinrichtung 2' dargestellt, mit welcher Licht, das von mit Licht durchstrahlten Proben in Wells 3 einer in diesen Mikroplatten-Reader 1 eingesetzten Mikroplatte 4 beeinflusst wird (also die Absorbance der Probe), gemessen werden kann. Diese erste Messeinrichtung 2' ist vorzugsweise im Probenraum 19 des Mikroplatten-Readers 1 angeordnet und eine erste Beleuchtungseinrichtung 11' zum Durchstrahlen der Proben in den Wells 3 einer Mikroplatte 4 ist bevorzugt im Geräteraum 18 des Mikroplatten-Readers 1 angeordnet. Dadurch ist die erste Messeinrichtung 2' zum Detektieren von Licht, das von Proben in Wells 3 einer in diesen Mikroplatten-Reader 1 eingesetzten Mikroplatte 4 beeinflusst wird, auf einer zweiten Seite 14 (also auf der Unterseite) einer in diesen Mikroplatten-Reader 1 eingesetzten Mikroplatte 4 und in Richtung einer ersten optischen Achse 13' angeordnet. Folglich ist die erste Beleuchtungseinrichtung 11' zum Durchstrahlen der Proben auf einer ersten Seite 12 (also auf der Oberseite) einer in diesen Mikroplatten-Reader 1 eingesetzten Mikroplatte 4 und ebenfalls in Richtung dieser ersten optischen Achse 13' angeordnet. Die Zuordnung der ersten Beleuchtungseinrichtung 11' zur ersten optischen Achse 13' erfolgt hier mit einem teilweise (z.B. 50%) durchlässigen Spiegel 26 oder mit einem Dichroidspiegel. Zu der ersten Beleuchtungseinrichtung 11' gehören ein erster wellenlängenselektiver Filter 37' und eine Blitzlampe 36. Somit wird hier das Licht der Blitzlampe 36 zum Durchleuchten der Proben mittels des teilweise durchlässigen Spiegels 26 in die Richtung der ersten optischen Achse 13' und via eine erste Optik 23' auf die Proben gelenkt. Diese erste Optik 23' kann in Richtung einer Z-Achse eines kartesischen Koordinatensystems höhenverschiebbar ausgebildet sein (vgl. Doppelpfeil Z). In diesem Fall ist zwischen der ersten Beleuchtungseinrichtung 11' und der ersten Messeinrichtung 2', jedoch bevorzugt im Probenraum 19, eine Aufnahmeeinrichtung 5 zur Aufnahme von zumindest einer Mikroplatte 4 und zum Positionieren der Proben enthaltenden Wells 3 dieser Mikroplatte(n) 4 gegenüber der ersten Messeinrichtung 2' und damit auch gegenüber der ersten optischen Achse 13' angeordnet.

In der Fig. 2 ist gemäss einer bevorzugten zweiten Variante des erfindungsgemässen Mikroplatten-Readers 1 eine zweite Messeinrichtung 2" dargestellt, mit welcher Licht, das von Proben in Wells 3 einer in diesen Mikroplatten-Reader 1 eingesetzten Mikroplatte 4 ausgesendet wird (also die Fluoreszenz der Probe), in Bezug auf die erste oder eine zweite optische Achse 13,'13" gemessen werden kann. Diese zweite Messeinrichtung 2" umfasst eine Fotomultiplier-Röhre (PMT) 24 und einen zweiten, wellenlängenselektiver Filter 37". Zu dieser Fluoreszenz-Messung wird wieder die erste Beleuchtungseinrichtung 11' des Mikroplatten-Readers 1 zum Bestrahlen (Anregen) der Proben in den Wells 3 einer Mikroplatte 4 verwendet. Die Zuordnung der ersten Beleuchtungseinrichtung 11' zur zweiten optischen Achse 13" erfolgt wieder mit dem teilweise (z.B. 50%) durchlässigen Spiegel 26 oder einem Dichroidspiegel und über die erste Optik 23'.

Zum Detektieren der von den Proben ausgehenden Fluoreszenz können zwei unterschiedliche Anordnungen verwendet werden:
- Beim sogenannten "Bottom Reading" befindet sich eine zweite Optik 23" unterhalb der Mikroplatte 4 und ist über eine erste faseroptische Leitung 33' mit dem teilweise durchlässigen Spiegel 26 und den zweiten Filter 37" verbunden, so dass die Fotomultiplier-Röhre (PMT) 24 zum Detektieren der Fluoreszenz jeder einzelnen Probe in einem Well 3 der Mikroplatte 4 verwendet werden kann.
- Beim sogenannten "Top Reading" wird die erste Optik 23' oberhalb der Mikroplatte 4 verwendet, welche über den teilweise durchlässigen Spiegel 26 mit dem zweiten Filter 37" verbunden ist, so dass dieselbe Fotomultiplier-Röhre (PMT) 24 zum Detektieren der Fluoreszenz jeder einzelnen Probe in einem Well 3 der Mikroplatte 4 verwendet werden kann.

In diesem Fall ist die Aufnahmeeinrichtung 5 zur Aufnahme von zumindest einer Mikroplatte 4 und zum Positionieren der Proben enthaltenden Wells 3 dieser Mikroplatte(n) 4 gegenüber der ersten oder zweiten Messeinrichtung 2',2" bevorzugt im Probenraum 19 und unterhalb der vorzugsweise im Geräteraum 18 untergebrachten ersten Beleuchtungseinrichtung 11', angeordnet.

In der Fig. 2 ist gemäss einer bevorzugten dritten Variante des erfindungsgemässen Mikroplatten-Readers 1 eine dritte Messeinrichtung 2''' dargestellt, mit welcher Licht, das von Proben in Wells 3 einer in diesen Mikroplatten-Reader 1 eingesetzten Mikroplatte 4 ausgesendet wird (also z.B. die Lumineszenz der Probe), gemessen werden kann. Zu dieser Lumineszenz-Messung wird eine, vorzugsweise im Geräteraum 18 des Mikroplatten-Readers 1 und in Bezug auf eine dritte optische Achse 13"' angeordnete, dritte Messeinrichtung 2'" verwendet. Zum Auslösen einer chemischen Reaktion, die mit einer Lumineszenzreaktion bzw. einer Veränderung der Lumineszenz, und/oder einer Veränderung der Fluoreszenz, und/oder einer Veränderung der Absorbance in oder an den Proben in den Wells 3 einer Mikroplatte 4 einhergehen kann, wird eine, vorzugsweise in den Probenraum 19 des Mikroplatten-Reader 1 reichende, an sich bekannte, Injektorvorrichtung 10 verwendet. Diese Injektorvorrichtung 10 ist vorzugsweise so gebaut und angeordnet, dass die z.B. die Lumineszenzreaktion auslösenden Reagenzien in ein Well 3 der Mikroplatte 4 zugegeben werden können, welches sich gerade in der dritten optischen Achse 13"' und damit gerade über der dritten Messeinrichtung 2"' befindet. Die dritte Messeinrichtung 2"' umfasst vorzugsweise eine dritte Optik 23"', die in Richtung einer Z-Achse eines kartesischen Koordinatensystems bewegbar ausgebildet sein kann (vgl. Doppelpfeil Z). Speziell bevorzugt sind die ersten und zweiten Optiken 23' und 23" jeweils durch eine Öffnung 20 in der Trennplatte 15 teilweise in den Probenraum 19 absenkbar ausgebildet.

Vorzugsweise wird bei allen bisher beschriebenen Varianten des erfindungsgemässen Mikroplatten-Readers 1 dieselbe Kontrolleinheit 6' verwendet (in der Fig. 2 der Einfachheit halber nicht dargestellt).

Figur 3 zeigt einen stark schematisierten Vertikalschnitt durch einen erfindungsgemässen Mikroplatten-Reader 1 gemäss einer bevorzugten dritten Ausführungsform mit einem Gehäuse 17, dessen Innenraum 16 mittels eines Innengehäuses 39 in einen Geräteraum 18 und in einen Probenraum 19 unterteilt ist. Dieser Mikroplatten-Reader 1 umfasst zumindest eine Messeinrichtung 2',2",2''' zum Detektieren von Licht. Das Innengehäuse 39 weist eine Öffnung 20 auf, die so ausgebildet ist, dass dadurch von Proben in Wells 3 der in den Mikroplatten-Reader 1 eingesetzten Mikroplatte 4 ausgesendetes oder beeinflusstes Licht vom Probenraum 19 in den Geräteraum 18 übertreten kann.

In der Fig. 3 ist gemäss einer bevorzugten ersten Variante des erfindungsgemässen Mikroplatten-Readers 1 eine erste Messeinrichtung 2' dargestellt, mit welcher Licht, das von mit Licht durchstrahlten Proben in Wells 3 einer in diesen Mikroplatten-Reader 1 eingesetzten Mikroplatte 4 beeinflusst wird (also die Absorbance der Probe), gemessen werden kann. Diese erste Messeinrichtung 2' ist vorzugsweise im Geräteraum 18 des Mikroplatten-Readers 1 angeordnet und eine erste Beleuchtungseinrichtung 11' zum Durchstrahlen der Proben in den Wells 3 einer Mikroplatte 4 ist bevorzugt im Probenraum des Mikroplatten-Readers 1 angeordnet. Dadurch ist die erste Messeinrichtung 2' zum Detektieren von Licht, das von Proben in Wells 3 einer in diesen Mikroplatten-Reader 1 eingesetzten Mikroplatte 4 beeinflusst wird, auf einer ersten Seite 12 (also auf der Oberseite) einer in diesen Mikroplatten-Reader 1 eingesetzten Mikroplatte 4 und in Richtung einer ersten optischen Achse 13' angeordnet. Folglich ist die erste Beleuchtungseinrichtung 11' zum Durchstrahlen der Proben auf einer zweiten Seite 14 (also auf der Unterseite) einer in diesen Mikroplatten-Reader 1 eingesetzten Mikroplatte 4 und ebenfalls in Richtung dieser ersten optischen Achse 13' angeordnet. Die erste Messeinrichtung 2' umfasst vorzugsweise einen Spiegel 25 zum Umlenken des von den Proben kommenden Lichts in Richtung einer Fotomultiplier-Röhre oder PMT 24. Alternativ kann das von den Proben kommende Licht mittels Faseroptik der PMT 24 zugeführt werden (nicht gezeigt). Gleichfalls kann die erste Beleuchtungseinrichtung 11' als Lampe ausgebildet und in der ersten optischen Achse 13' angeordnet sein und alternativ kann das Licht zum Durchleuchten der Proben mittels Spiegel oder Faseroptik (beides nicht gezeigt) in die Richtung der ersten optischen Achse 13' gelenkt werden. In diesem Fall ist zwischen der ersten Beleuchtungseinrichtung 11' und der ersten Messeinrichtung 2', jedoch bevorzugt im Probenraum 19, eine Aufnahmeeinrichtung 5 zur Aufnahme von zumindest einer Mikroplatte 4 und zum Positionieren der Proben enthaltenden Wells 3 dieser Mikroplatte(n) 4 gegenüber der ersten Messeinrichtung 2' angeordnet.

In der Fig. 3 ist gemäss einer bevorzugten zweiten Variante des erfindungsgemässen Mikroplatten-Readers 1 eine zweite Messeinrichtung 2" dargestellt, mit welcher Licht, das von Proben in Wells 3 einer in diesen Mikroplatten-Reader 1 eingesetzten Mikroplatte 4 ausgesendet wird (also die Fluoreszenz der Probe), gemessen werden kann. Zu dieser Fluoreszenz-Messung wird wieder die erste Messeinrichtung 2' des Mikroplatten-Readers 1 verwendet, welche hier mit der zweiten Messeinrichtung 2" identisch ist. Als Quelle für das dafür erforderliche Anregungslicht wird eine zweite Beleuchtungseinrichtung 11" (vorzugsweise ein Laser, eine Blitzlampe oder eine Laserdiode) zum Bestrahlen der Proben in den Wells 3 einer Mikroplatte 4 verwendet. Diese zweite Beleuchtungseinrichtung 11" ist bevorzugt im Geräteraum 18 des Mikroplatten-Readers 1 angeordnet. Dadurch befinden sich sowohl die erste Messeinrichtung 2' zum Detektieren von Licht, das von Proben in Wells 3 einer in diesen Mikroplatten-Reader 1 eingesetzten Mikroplatte 4 ausgesendet wird, als auch die zweite Beleuchtungseinrichtung 11" auf der gleichen Seite 12 (also auf der Oberseite) einer in diesen Mikroplatten-Reader 1 eingesetzten Mikroplatte 4. Die zweite Beleuchtungseinrichtung 11" umfasst vorzugsweise einen teilweise (z.B. 50%) durchlässigen Spiegel 26 oder einen Dichroidspiegel zum Umlenken des Anregungslichts in Richtung der ersten optischen Achse 13' und damit auf bzw. in die Proben. In diesem Fall ist die Aufnahmeeinrichtung 5 zur Aufnahme von zumindest einer Mikroplatte 4 und zum Positionieren der Proben enthaltenden Wells 3 dieser Mikroplatte(n) 4 gegenüber der ersten Messeinrichtung 2' bevorzugt im Probenraum 19 und unterhalb der vorzugsweise im Geräteraum 18 untergebrachten zweiten Beleuchtungseinrichtung 11' und ersten Messeinrichtung 2', angeordnet.

In der Fig. 3 ist gemäss einer bevorzugten dritten Variante des erfindungsgemässen Mikroplatten-Readers 1 eine dritte Messeinrichtung 2'" dargestellt, mit welcher Licht, das von Proben in Wells 3 einer in diesen Mikroplatten-Reader 1 eingesetzten Mikroplatte 4 ausgesendet wird (also die Lumineszenz der Probe), gemessen werden kann. Zu dieser Lumineszenz-Messung wird eine, vorzugsweise im Probenraum 19 des Mikroplatten-Readers 1 und in Bezug auf eine zweite optische Achse 13" angeordnete, dritte Messeinrichtung 2'" verwendet. Zum Auslösen einer Lumineszenzreaktion oder einer anderen chemischen Reaktion in oder an den Proben in den Wells 3 einer Mikroplatte 4 wird eine, vorzugsweise im Probenraum 19 des Mikroplatten-Reader 1 angeordnete, an sich bekannte, Injektorvorrichtung 10 verwendet. Diese Injektorvorrichtung 10 ist beispielsweise so gebaut und angeordnet, dass die die Lumineszenzreaktion auslösenden Reagenzien in ein Well 3 der Mikroplatte 4 zugegeben werden können, welches sich gerade in der zweiten optischen Achse 13" und damit gerade über der dritten Messeinrichtung 2''' befindet. In diesem Fall ist die Aufnahmeeinrichtung 5 zur Aufnahme von zumindest einer Mikroplatte 4 und zum Positionieren der Proben enthaltenden Wells 3 dieser Mikroplatte(n) 4 gegenüber der zweiten Messeinrichtung 2" bevorzugt im Probenraum 19, unterhalb der Injektorvorrichtung 10 und über der dritten Messeinrichtung 2'" angeordnet. Die erste Messeinrichtung 2' umfasst vorzugsweise eine erste Optik 23', die in Richtung einer Z-Achse eines kartesischen Koordinatensystems bewegbar ausgebildet sein kann. Speziell bevorzugt, entsprechend der in der Fig. 3 gezeigten ersten Ausführung, ist die erste Optik 23' durch eine Öffnung 20 in dem Innengehäuse 39 teilweise in den Probenraum 19 absenkbar ausgebildet.

Die Mikroplatte 4 bzw. die Aufnahmeeinrichtung 5 ist relativ zu der ersten, zweiten bzw. dritten Optik 23', 23", 23"' der ersten, zweiten, bzw. dritten Messeinrichtung 2', 2", 2'" in Richtung einer Z-Achse eines kartesischen Koordinatensystems (vgl. Doppelpfeil Z in Fig. 2 und 3) bewegbar. Die Mikroplatte 4 bzw. die Aufnahmeeinrichtung 5 (vgl. Doppelpfeil X, Y) und/oder die erste, zweite, bzw. dritte Messeinrichtung 2', 2", 2"' bzw. deren erste, zweite bzw. dritte Optik 23', 23", 23"' können auch in Richtung einer X-Achse und/oder einer Y-Achse des kartesischen Koordinatensystems bewegbar ausgebildet sein. Dies gilt für alle gezeigten bevorzugten Ausführungsformen und Varianten des erfindungsgemässen Mikroplatten-Readers 1 und etwaige Abwandlungen davon.

Alle Ausführungsformen und Varianten des erfindungsgemässen Mikroplatten-Readers 1 sind dadurch gekennzeichnet, dass der Mikroplatten-Reader 1 eine Kontrolleinheit 6 zur Steuerung der Zusammensetzung einer Gasatmosphäre 7 um die den Proben enthaltenden Wells 3 von in diesen Mikroplatten-Reader 1 eingesetzten Mikroplatten 4 umfasst. Zudem ist in jedem Fall die Aufnahmeeinrichtung 5 bevorzugt in Richtung einer X- und Y-Achse eines kartesischen Koordinatensystems bewegbar ausgebildet (vgl. Fig. 1-3: Doppelpfeile X,Y).

Die Figur 4 zeigt eine beispielhafte Anordnung eines O₂-Sensors 8, eines CO₂-Sensors 9, eines Lüfters 22 und eines Gaseinlasses 21 an der Innenseite der Rückwand 30 des Gehäuses 17 eines bevorzugten erfindungsgemässen Mikroplatten-Readers 1. Die Kontrolleinheit 6 des Mikroplatten-Readers 1 umfasst bevorzugt einen O₂-Sensor 8 zur Messung und Steuerung des Sauerstoffgehalts der Gasatmosphäre 7 um die den Proben enthaltenden Wells 3 von in diesen Mikroplatten-Reader 1 eingesetzten Mikroplatten 4. Besonders bevorzugt umfasst die Kontrolleinheit 6 des Mikroplatten-Readers 1 alternativ oder ergänzend zu diesem O₂-Sensor 8 einen CO₂-Sensor 9 zur Messung und Steuerung des Kohlendioxidgehalts der Gasatmosphäre 7 um die den Proben enthaltenden Wells 3 von in diesen Mikroplatten-Reader 1 eingesetzten Mikroplatte(n) 4. Diese Kontrolleinheit 6 kann teilweise ausserhalb des Gehäuses 17 des Mikroplatten-Readers 1 angeordnet und beispielsweise über elektrische Kontakte 27 und Gasleitungen (nicht gezeigt) mit dem Mikroplatten-Reader 1 verbunden bzw. verbindbar sein (nicht gezeigt). Es kann aber auch vorgesehen werden, dass die Kontrolleinheit 6 vollständig in den Mikroplatten-Reader 1 integriert und einem gemeinsamen Gehäuse 17 untergebracht ist (vgl. Fig. 2 und 3); in diesem Fall würde der Mikroplatten-Reader 1 direkt an die notwendigen Druckflaschen für die zu verwendenden Gase angeschlossen (vgl. Fig. 2 und 3). Vorzugsweise sind dann die entsprechenden Ventile und Drosseln für die benötigten Gasanschlüsse ebenfalls in das Gehäuse 17 des Mikroplatten-Readers 1 eingebaut (nicht gezeigt). Alternativ können auch die Ventile und Drosseln auf den Gasdruckflaschen verwendet werden, wobei diese Ventile vorzugsweise als elektrisch gesteuerte Magnetventile (des Typs stromlos geschlossen) ausgebildet sind (nicht gezeigt).

Die Kontrolleinheit umfasst vorzugsweise einen Rechner 28 mit der entsprechenden Software; dabei kann der Rechner 28 mit einem zentralen Rechner 29 des Mikroplatten-Readers 1 verbunden (vgl. Fig. 1), verbindbar (z.B. in einem separaten Gehäuse 17' untergebracht) oder in diesen zentralen Rechner 29 des Mikroplatten-Readers 1 integriert sein (nicht gezeigt).

Vorzugsweise werden der zum Verdrängen der Umgebungsluft verwendete Stickstoff (N₂) und/oder das zu verwendende Kohlendioxyd (CO₂) in Druckflaschen bereitgestellt, wobei an sich bekannte Regelventile und Drosseln an diesen Druckflaschen verwendet werden, um den benötigten Lieferdruck für diese Gase einzustellen. Alternativ können derartige Prozessgase auch aus anderen Quellen (z.B. aus Hausleitungen) bezogen werden. Neben Stickstoff und Kohlendioxyd können (kombiniert mit entsprechenden Gasdetektoren im Probenraum 19) auch andere Gase zum Erzeugen eines definierten Anteils an der normalerweise im Probenraum vorherrschenden Atmosphäre eingesetzt werden. Bei Beachtung der unter Umständen geltenden Vorsichtsmassnahmen können somit auch andere Gase, wie beispielsweise Edelgase bzw. inerte Gase (z.B. Argon) oder auch reaktive oder giftige Gase (z.B. Sauerstoff, Kohlenmonoxid, Schwefelwasserstoff oder Schwefeldioxid) zum Erzeugen einer bestimmten Zusammensetzung der Gasatmosphäre über den bzw. in der Umgebung der Wells 3 einer in diesen Mikroplatten-Reader 1 eingesetzten Mikroplatte 4 über mindestens einen Gaseinlass 21 in den Probenraum 19 des Mikroplatten-Readers 1 eingeleitet werden. Vorzugsweise ist die Kontrolleinheit 6 mit genügend Gaszuleitungen und Steuerventilen ausgerüstet, so dass auch komplexere Gaszusammensetzungen mit mehreren Gas-Komponenten ermöglicht werden.

Stellvertretend für geeignete CO₂-Sensoren soll der aktuell verwendete CO₂-Sensor SenseAir^{®} CO₂ Engine^{®} ICB, Part No.: 033-9-0001 der Firma SenseAir AB in SE-820 60 Delsbo, Schweden, genannt werden. Stellvertretend für geeignete O₂-Sensoren soll der aktuell verwendete O₂-Sensor Pewatron FCX-MEP2-F-CH Sauerstoffmodul der Firma Pewatron AG in CH-8052 Zürich, Schweiz, genannt werden.

Die Kontrolleinheit 6 in Kombination mit einem O₂-Sensor ermöglicht somit das Steuern des Sauerstoffgehalts der Gasatmosphäre 7 um die den Proben enthaltenden Wells 3 dieser in diesen Mikroplatten-Reader 1 eingesetzten Mikroplatte(n) 4, so dass dieser Mikroplatten-Reader 1 beim Vermessen mikroaerophiler oder fakultativ anaerober Mikroorganismen bei einer definierten O₂-Konzentration verwendet werden kann. Auch das Vermessen anaerober Mikroorganismen oder eukaryotischer Zellen bei einer definierten O₂-Konzentration ist somit möglich.

Die Kontrolleinheit 6 in Kombination mit einem CO₂-Sensor ermöglicht somit das Steuern des Kohlendioxidgehalts der Gasatmosphäre 7 um die den Proben enthaltenden Wells 3 dieser in diesen Mikroplatten-Reader 1 eingesetzten Mikroplatte(n) 4, so dass dieser Mikroplatten-Reader 1 beim Vermessen von lebenden Zellkulturen bei einer definierten CO₂-Konzentration verwendet werden kann.

Im Zusammenhang mit der vorliegenden Erfindung werden Zellkulturen, von solchen abgetrennte oder sonstwie gewonnene biologische Zellansammlungen oder Einzelzellen als Zellen bezeichnet, wobei diese Zellen Mikroorganismen und Pilze sowie tierische und pflanzliche Eukaryotenzellen umfassen.

Beliebige Kombinationen der in den Figuren 1 bis 4 gezeigten Elemente des bevorzugten Mikroplatten-Readers 1 und der Kontrolleinheit 6 gehören zum Umfang der vorliegenden Erfindung. Der Umfang wird durch die Ansprüche definiert.

Mit einem erfindungsgemässen Mikroplatten-Reader 1 der ersten bevorzugten Ausführungsform (vgl. Fig. 1) wurde die 5% CO₂-Regulation an eukaryotischen Tumorzellen getestet. Es wurden für die Fluoreszenzbestimmung GFP-transfizierte A431 Zellen verwendet. Dabei bezeichnet die Abkürzung GFP das an sich bekannte "grün fluoreszierende Protein" mit Anregungsmaxima bei einer Wellenlänge von 396 nm und 475 nm und mit einem Emissionsmaximum bei einer Wellenlänge von 508 nm. Zur Aufnahme der entsprechenden Wachstumskurven über 72 Stunden wurden diese Zellen in Medien mit 10% Serum (MMS) oder in Medien ohne Serum (MOS) suspendiert.

Das Medium mit Serum (MMS) hatte die folgende Zusammensetzung: Dulbecco's modified Eagle's Medium (DMEM enthaltend Phenolrot) mit 4.5 g/l Glukose ergänzt mit 10 mM HEPES Puffer, 2 mM L-Glutamine, 1 mM Na-Pyruvat, 100 U/ml Penicillin, 0.1 mg/ml Streptomycin und 5% (v/v) fötalem Rinderserum (FBS). Alle Medien-Komponenten wurden von PAA-Laboratorien, Linz, Österreich bezogen. Das Medium ohne Serum (MOS) enthielt die gleichen Komponenten gleich, jedoch kein fötales Rinderserum (FBS).

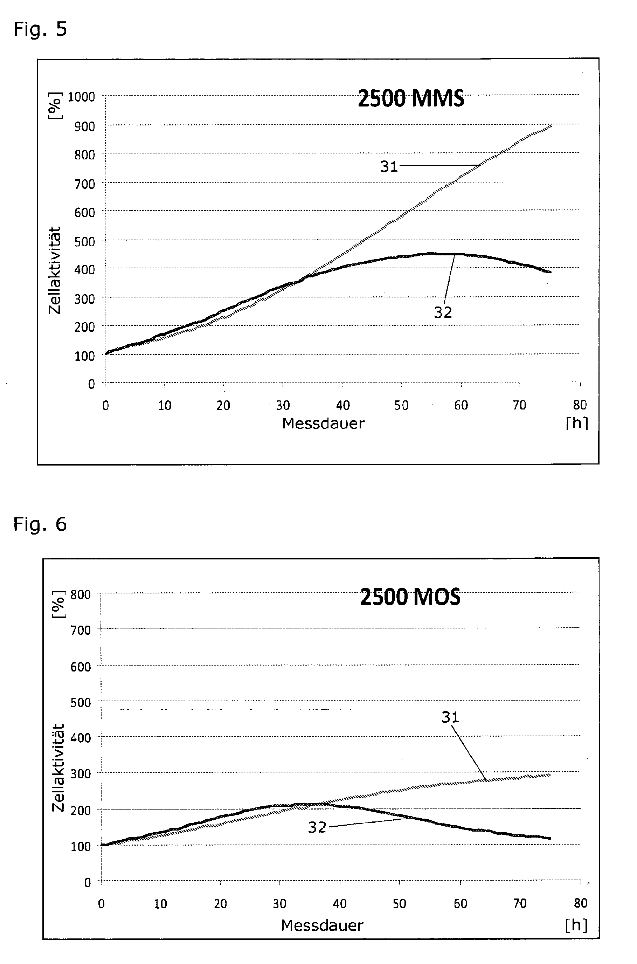

Es wurden Mikroplatten 4 des Typs "Greiner Standard Zellkultur MTP 96 Well steril mit Beschichtung (Greiner 96 well, black, flat & clear bottom, steril - culture tissue plates) verwendet. 48 Wells 3 dieser Mikroplatten 4 wurden mit jeweils 2500 A431GFP-Zellen und mit 10% Serum beschickt (Probentyp A vgl. Fig. 5). Die restlichen 48 Wells dieser Mikroplatten 4 wurden mit jeweils 2500 A431GFP-Zellen ohne Serum beschickt (Probentyp B vgl. Fig. 6). Zwei Versuchsreihen mit diesen beiden Probentypen A und B wurde durchgeführt:

Eine erste Versuchsreihe bestand darin, dass mit einem CO₂-Sensor (SenseAir^{®}, Typ CO₂ Engine^{®} ICB, Part No.: 033-9-0001) die CO₂-Konzentration im Probenraum 19 gemessen und mit einer Kontrolleinheit 6 mit integrierter Steuerelektronik konstant auf 5% gehalten wurde. Dies wurde dadurch erreicht, dass die Atmosphäre im Probenraum 19 und im Geräteraum 18 des Mikroplatten-Readers 1 gemäss eines bevorzugten Ausführungbeispiel der Erfindung anfänglich eine Gaszusammensetzung aufwies, welche derjenigen der Umgebungsluft, d.h. in etwa der Standardatmosphäre entsprach, welche die folgende Zusammensetzung aufweist: Sauerstoff 20,93%; Stickstoff 78,10%; Argon 0,93%; Kohlendioxyd 0,03%; Wasserstoff, Neon, Helium, Krypton und Xenon 0,01%. Nach dem Platzieren der Mikroplatte 4 auf der Aufnahmeeinrichtung 5 des Mikroplatten-Readers 1 wurde das Gehäuse 17 des Mikroplatten-Readers 1 geschlossen und über den Gaseinlass 21 wurde CO₂-Gas in den Probenraum 18 eingelassen, bis die CO₂-Konzentration 5% betrug. Über die Dauer der Messung wurde diese CO₂-Konzentration permanent auf 5% gehalten, indem CO₂-Gas nach Erfordernis (sobald der CO₂-Sensor eine zu tiefe Kohlendioxidkonzentration feststellte) in den Probenraum 19 eingelassen wurde. Mit dem Lüfter 22 wurde das Gasgemisch im Probenraum 19 während den Versuchsreihen konstant umgewälzt. Die Temperatur wurde in jedem Fall konstant auf 37 °C gehalten.

Eine zweite Versuchsreihe bestand darin, dass kein zusätzliches CO₂ in den Probenraum 19 eingelassen und die Proben damit dauernd der Umgebungsluft ausgesetzt waren. Die Temperatur wurde in jedem Fall konstant auf 37 °C gehalten.

Die Fluoreszenz jeder Probe (jedes Wells 3) wurde jeweils mit einer ersten, unter der Mikroplatte 4 angeordneten Beleuchtungseinrichtung 11' angeregt (Anregung bei λ=485 nm; Fluoreszenz-Detektion bei λ=535 nm) und mittels der zweiten Optik 23" an eine Fotomultiplier-Röhre 24 zur Detektion geleitet. Gemessen wurde die individuelle Fluoreszenz der einzelnen Proben in Abhängigkeit der Zeit. Die von der Fotomultiplier-Röhre 24 detektierten individuellen Intensitäten der Fluoreszenz jedes einzelnen Wells wurden im zentralen Rechner 29 des Mikroplatten-Readers 1 verarbeitet und dann in Kurvendiagrammen dargestellt (vgl. Fig. 5 und 6).

Die Figur 5 zeigt unter Verwendung eines Mikroplatten-Readers 1 gemäss eines bevorzugten Ausführungbeispiel der Erfindung mit oder ohne CO₂-Regelung erstellte Wachstumskurven von eukaryotischen Tumorzellen in einem Medium mit 10% Serum (MMS). Dabei bezeichnet das Bezugszeichen 31 die Proben, bei denen die CO₂-Konzentration permanent auf 5% gehalten wurde und das Bezugszeichen 32 die Proben, die der normalen Umgebungsluft ausgesetzt waren. Aus der Fig. 5 geht hervor, dass sich die Zellen während den ersten 35 Stunden in etwa gleich verhielten, wobei die Zellvitalität (gemessen an Hand des korrespondierenden GFP-Signals (also an Hand der Fluoreszenz-Intensivität) der Proben 31, bei denen die CO₂-Konzentration permanent auf 5% gehalten wurde, dauernd etwas niedriger war als die Zellvitalität der Proben 32, die der normalen Umgebungsluft ausgesetzt waren. Nach etwa 35 Stunden verlangsamte sich das Wachstum der Zellen der Proben 32, die der normalen Umgebungsluft ausgesetzt waren dramatisch; dieses erreichte nach etwa 60 Stunden ein Maximum von ca. 450% und sank bis zum Ende der Messung bei 75 Stunden auf unter 400% des anfänglichen Wertes. Im Gegensatz dazu nahm die Zellvitalität der Proben 31, bei denen die CO₂-Konzentration permanent auf 5% gehalten wurde, nach etwa 35 Stunden Messdauer praktisch linear zu und erreichte bis zum Ende der Messung bei 75 Stunden etwa 900% des anfänglichen Wertes.

Die Figur 6 zeigt unter Verwendung eines Mikroplatten-Readers 1 gemäss eines bevorzugten Ausführungbeispiel der Erfindung mit oder ohne CO₂-Regelung erstellte Wachstumskurven von eukaryotischen Tumorzellen in einem Medium ohne Serum (MOS). Dabei bezeichnet das Bezugszeichen 31 die Proben, bei denen die CO₂-Konzentration permanent auf 5% gehalten wurde und das Bezugszeichen 32 die Proben, die der normalen Umgebungsluft ausgesetzt waren. Aus der Fig. 6 geht hervor, dass sich die Zellen während den ersten 35 Stunden in etwa gleich verhielten, wobei die Zellvitalität (gemessen an Hand des korrespondierenden GFP-Signals (also an Hand der Fluoreszenz-Intensivität) der Proben 31, bei denen die CO₂-Konzentration permanent auf 5% gehalten wurde, dauernd etwas niedriger war als die Zellvitalität der Proben 32, die der normalen Umgebungsluft ausgesetzt waren. Bei etwa 35 Stunden erreichte das Wachstum der Zellen der Proben 32, die der normalen Umgebungsluft ausgesetzt waren ein Maximum von ca. 200% und sank bis zum Ende der Messung bei 75 Stunden auf ungefähr den anfänglichen Wert von 100%. Im Gegensatz dazu nahm die Zellvitalität der Proben 31, bei denen die CO₂-Konzentration permanent auf 5% gehalten wurde, auch nach etwa 35 Stunden Messdauer praktisch in gleichem Masse zu und erreichte bis zum Ende der Messung bei 75 Stunden beinahe 300% des anfänglichen Wertes.

Aus den gezeigten Resultaten ergibt sich, dass die permanent bei einer CO₂-Konzentration von 5% gehaltenen Zellen eine wesentlich grössere Zellvitalität zeigen können als Zellen, die bei normaler Umgebungsluft gehalten werden. Diese grössere Zellvitalität spielt vor allem nach einer Inkubationszeit von mehr als 40 Stunden eine Rolle. Die zusätzliche Zugabe von 10% Serum steigert die Zellvitalität während der ersten 35 Stunden um ca. einen Faktor 2, nach 75 Stunden sogar um einen Faktor 3.

Im Gegensatz zu herkömmlichen Inkubatoren ohne integrierte Fluoreszenz-Messung werden solche ununterbrochenen Langzeitstudien überhaupt möglich, weil mit der Verwendung von herkömmlichen CO₂-Inkubatoren typischerweise ein Nachtfenster von ca. 14 Stunden in Kauf genommen werden muss. Ein Nachtfenster, in welchem keine Daten erfasst werden können.

Aus dem Stand der Technik sind keine Mikroplatten-Reader bekannt, die im Probenraum angeordnete Gas-Sensoren und eine Kontrolleinheit zur Steuerung der Zusammensetzung der Gasatmosphäre in diesem Probenraum über den bzw. in der Umgebung der Proben enthaltenden Wells einer in diesen Mikroplatten-Reader eingesetzten Mikroplatte umfassen.

Aus den Dokumenten US 2006/0023299 A1, oder US 2005/0196325 A1, oder US 2008/0180793 A1, oder US 6,548,263 B1 sind gattungsgemässe Mikroplatten-Reader mit zumindest einer Fluoreszenz-, oder Absorbance-, oder Fluoreszenz- Messeinrichtung für die mit Licht angestrahlten Proben in Wells einer in der Mikroplatten-Reader eingesetzten Mikroplatte; mit einer Mikroplatten-Aufnahmeeinrichtung zum Positionieren der Proben enthaltenden Wells dieser Mikroplatte gegenüber einer dieser Messeinrichtungen; mit einer Kontrolleinheit zur Steuerung der Zusammensetzung der Gasatmosphäre um die Proben enthaltenden Wells sowie mit einem Begrenzungsmittel in Form einer Trennplatte oder eines Innengehäuses bekannt.

Aus den Dokumenten WO 2010/0110670 A1 und EP 2 325 326 A1 sind Reader gemäss des Oberbegriffs des Anspruchs 1 bekannt. Der offenbarte Reader wird jedoch verwendet zur Analyse von Kulturschalen und nicht von Mikroplatten.

Die deutsche Offenlegungsschrift DE 10 2005 033 927 A1 offenbart zwar eine Beleuchtungseinrichtung für einen Durchlichtkontrast im Hellfeld für inverse Mikroskope zur Lebendzellbeobachtung. Diese Beleuchtungseinrichtung ist in einen hermetisch abgeschlossenen, lichtdichten und kompakten Inkubator des inversen Mikroskops so integriert, dass jedes einzelne Well eines als Mikrotiterplatte ausgebildeten Probenträgers nacheinander ausgeleuchtet wird, und dass gleichzeitig eine thermische Stabilisierung des Probenvolumens über einen langen Zeitraum bei möglichst geringen Wärmeverlusten gewährleistet wird. Diese Beleuchtungseinrichtung ist ortsfest in einem Inkubatoroberteil und über einer Abbildungsoptik angeordnet. Eine eingesetzte Mikrotiterplatte, Petrischale oder dergleichen ist auf einem in einem Unterteil des Inkubators angeordneten Mikroskoptisch relativ zur Abbildungsoptik bewegbar. Der Inkubator weist eine erste innere Kammer auf, die von einer äusseren zweiten Kammer vollständig umhüllt und dadurch von der Umgebung thermisch entkoppelt ist. Die Mikrotiterplatte mit den Proben befindet sich in dieser inneren Kammer, in welcher die Temperatur, die Luftfeuchte und der CO₂-Gehalt durch Steuergeräte kontrolliert steuerbar sind. Diese deutsche Offenlegungsschrift DE 10 2005 033 927 A1 bezieht sich ausschliesslich auf inverse Mikroskope, deren Aufbau und Verwendung sich erheblich von einem Mikroplatten-Reader unterscheiden, weil dieser keine bildgebende Funktion umfasst und wesentlich kürzere Messzeiten pro Well ermöglicht. Mikroplatten-Reader und insbesondere auch O₂-Sensoren werden in dieser Publikation nicht erwähnt.

Auch aus dem Patent EP 1 575 706 B1 ist eine Klimakammer für Mikroskope zum Beobachten von Proben in Mikrotiterplatten bekannt. In diese Klimakammer kann ein klimatisierender Medienstrom in Form von Luft mit einer definierten Luftfeuch-tigkeit und/oder Temperatur eingeleitet werden. Auch das definierte Einleiten von CO₂-Gas und das Anordnen eines Temperaturfühlers, Feuchtigkeitssensors und/oder Gassensors in der Nähe eines die Mikrotiterplatte tragenden Probenträgers können vorgesehen sein. Mikroplatten-Reader und insbesondere auch O₂-Sensoren werden in dieser Publikation nicht erwähnt.

Somit legt der Stand der Technik einem Fachmann den erfindungsgemässen Mikroplatten-Reader 1 gemäss Anspruch 1 mit der Kontrolleinheit 6, die im Probenraum 19 angeordnete Gassensoren zum Messen der Konzentration zumindest eines Gases zur Steuerung der Zusammensetzung einer Gasatmosphäre 7 über den bzw. in der Umgebung der Proben enthaltenden Wells 3 von in diesen Mikroplatten-Reader 1 eingesetzten Mikroplatten 4 umfasst, nicht nahe. Noch weniger legt der bekannte Stand der Technik einem Fachmann nahe, im Probenraum 19 zumindest eine Bewegungseinrichtung zum Mischen und/oder Umwälzen von im Probenraum 19 anwesenden Gasen anzuordnen.

Weitere bevorzugte Anwendungen des erfindungsgemässen Mikroplatten-Readers 1 und der Kontrolleinheit 6 betreffen beispielsweise Untersuchungen in Bezug auf den Einfluss des O₂-Partialdrucks auf das Wachstum von Mikroorganismen wie *Rhodospirillum rubrum* [Biedermann et al. 1967, Archiv für Mikrobiologie 56, 133-147] und Untersuchungen zur mikroaerophilen Alginatproduktion, bei der vorzugsweise eine O₂-Konzentration von 2.5-5% (vorzugsweise 2.5%) eingestellt werden sollte [Wael Sabra 1999: Mikroaerophile Alginatproduktion mit Azotobacter vinelandii; Dissertation an der Gemeinsamen Naturwissenschaftlichen Fakultät der Technischen Universität Carolo-Wilhemina zu Braunschweig].

In den Figuren gezeigte gleiche bzw. entsprechende Merkmale des bevorzugten erfindungsgemässen Mikroplatten-Readers 1 sind mit gleichen Bezugszeichen versehen, auch wenn in der Beschreibung dazu nicht ausdrücklich Bezug genommen wird. Zum Umfang der durch die beigefügten Patentansprüche definierten Erfindung gehören auch beliebige Kombinationen von Merkmalen der in den Figuren dargestellten Ausführungsformen.

### Bezugszeichenliste:

- 1: Mikroplatten-Reader
- 2': erste Messeinrichtung
- 2": zweite Messeinrichtung
- 2''': dritte Messeinrichtung
- 3: Wells
- 4: Mikroplatte
- 5: Aufnahmeeinrichtung
- 6: Kontrolleinheit
- 6': separate Kontrolleinheit
- 7: Gasatmosphäre
- 8: O₂-Sensor
- 9: CO₂-Sensor
- 10: Injektorvorrichtung
- 11': erste Beleuchtungseinrichtung
- 11": zweite Beleuchtungseinrichtung
- 12: erste Seite einer eingesetzten Mikroplatte
- 13': erste optische Achse
- 13": zweite optische Achse
- 13"': dritte optische Achse
- 14: zweite Seite einer eingesetzten Mikroplatte
- 15: Trennplatte
- 16: Innenraum
- 17: Gehäuse des Readers
- 17': separates Gehäuse
- 18: Geräteraum
- 19: Probenraum
- 20: Öffnung
- 21: Gaseinlass
- 22: Lüfter
- 23': erste Optik
- 23": zweite Optik
- 23''': dritte Optik
- 24: PMT (Photo Multiplier Tube) Fotomultiplier-Röhre
- 25: Spiegel
- 26: teilweise durchlässiger Spiegel
- 27: elektrische Kontakte
- 28: Rechner
- 29: zentraler Rechner Mikroplatten-Reader
- 30: Rückwand
- 31: Wachstumskurve von Zellen mit CO₂-Regelung
- 32: Wachstumskurve von Zellen ohne CO₂-Regelung
- 33': erste faseroptische Leitung
- 33": zweite faseroptische Leitung
- 33"': dritte faseroptische Leitung
- 34': erster Faserschieber
- 34": zweiter Faserschieber
- 35': erster Monochromator
- 35": zweiter Monochromator
- 36: Blitzlampe
- 37': erster Filter
- 37": zweiter Filter
- 38: Gasleitungen
- 39: Innengehäuse

## Patentansprüche

1. Mikroplatten-Reader (1) umfassend:
a) wenigstens eine Messeinrichtung (2',2",2"'), ausgewählt aus einer Gruppe umfassend:
a1) eine erste Messeinrichtung (2'), die zum Messen der Absorbance von mit Licht einer ersten Beleuchtungseinrichtung (11') durchstrahlten Proben in Wells (3) einer in den Mikroplatten-Reader (1) eingesetzten Mikroplatte (4) ausgebildet ist,
a2) eine zweite Messeinrichtung (2"), die zum Messen der Fluoreszenz von mit Licht einer ersten Beleuchtungseinrichtung (11') angestrahlten Proben in Wells (3) einer in den Mikroplatten-Reader (1) eingesetzten Mikroplatte (4) ausgebildet ist, und
a3) eine dritte Messeinrichtung (2"'), die zum Messen der Lumineszenz von Proben in Wells (3) einer in den Mikroplatten-Reader (1) eingesetzten Mikroplatte (4) ausgebildet ist;
b) eine Aufnahmeeinrichtung (5), die zur Aufnahme von zumindest einer Mikroplatte (4) während dem Messen mit zumindest einer der Messeinrichtungen (2',2",2"') ausgebildet ist;
c) ein Begrenzungsmittel, alternativ in Form einer Trennplatte (15) oder eines Innengehäuses (39), welches einen Innenraum (16) eines Gehäuses (17) des Mikroplatten-Readers (1) in einen Probenraum (19) mit einer die Proben enthaltenden Wells (3) einer eingesetzten Mikroplatte (4) umgebenden Gasatmosphäre (7) und einen Geräteraum (18) unterteilt, wobei der Probenraum (19) im Wesentlichen gasdicht von dem Geräteraum abgetrennt ist, und wobei das Begrenzungsmittel unbewegbar in dem Gehäuse (17) angeordnet ist und mindestens eine Öffnung (20) umfasst, die so ausgebildet ist, dass dadurch das von Proben in Wells (3) einer in den Mikroplatten-Reader (1) eingesetzten Mikroplatte (4) ausgesendete oder beeinflusste Licht vom Probenraum (19) in den Geräteraum (18) übertreten kann; und
d) eine Kontrolleinheit (6,6') zur Steuerung der Zusammensetzung einer Gasatmosphäre (7) im Probenraum (19), wobei die Kontrolleinheit (6,6') im Probenraum (19) angeordnete Gassensoren zum Messen der Konzentration zumindest eines Gases der Gasatmosphäre (7) im Probenraum (19) umfasst;
**dadurch gekennzeichnet, dass** der Mikroplatten-Reader (1) zudem zumindest eine erste Beleuchtungseinrichtung (11') mit einer Blitzlampe (36), einer ersten faseroptischen Leitung (33') und einem ersten, Wellenlängen selektierenden Monochromator (35') umfasst, wobei diese erste Beleuchtungseinrichtung (11') zum Durchstrahlen bzw. zum Anstrahlen von Proben in Wells (3) einer in den Mikroplatten-Reader (1) eingesetzten Mikroplatte (4) mit Licht ausgewählter Wellenlängen ausgelegt ist, wobei die erste Beleuchtungseinrichtung (11') des Mikroplatten-Readers (1) zumindest eine erste Optik (23') umfasst, die in der Öffnung (20) in der Trennplatte (15) bzw. in einer Wand des Innengehäuses (39) angeordnet ist;
und dass die Aufnahmeeinrichtung (5) zudem zum Positionieren der Proben enthaltenden Wells (3) dieser zumindest einen Mikroplatte (4) gegenüber der zumindest einen Messeinrichtung (2',2'',2''') zumindest in Richtung einer Achse eines kartesischen Koordinatensystems bewegbar ausgebildet und vollständig in dem Probenraum (19) angeordnet und von der Gasatmosphäre des Probenraums (19) umgeben ist.

2. Mikroplatten-Reader (1) nach Anspruch 1, **dadurch gekennzeichnet, dass** mittels der Trennplatte (15) bzw. des Innengehäuses der Probenraum (19) von dem Geräteraum (18) zudem im Wesentlichen lichtdicht abgetrennt ist, wobei zum lichtdichten und/oder gasdichten Abtrennen vorzugsweise eine sich um die Öffnung (20) herum erstreckende Abdichteinrichtung vorgesehen ist.

3. Mikroplatten-Reader (1) nach Anspruch 1 oder 2, **dadurch gekennzeichnet, dass** in dem Probenraum (19) eine Bewegungseinrichtung zum Mischen und/oder Umwälzen von in dem Probenraum (19) anwesendem und/oder einströmendem Gas angeordnet ist.

4. Mikroplatten-Reader (1) nach Anspruch 3, **dadurch gekennzeichnet, dass** die Bewegungseinrichtung mindestens eine der folgenden Einrichtungen umfasst: einen Lüfter (22), eine Einrichtung mit einer oder mehreren Prallplatten, eine Schütteleinrichtung mit einem oder mehreren Paddeln oder ein strukturiertes Düsensystem.

5. Mikroplatten-Reader (1) nach einem der Ansprüche 1 bis 4, **dadurch gekennzeichnet, dass** die Kontrolleinheit (6,6') einen Rechner (28) mit entsprechender Software umfasst, wobei der Rechner (28) mit einem zentralen Rechner (29) des Mikroplatten-Readers (1) verbindbar oder in diesen integriert ist.

6. Mikroplatten-Reader (1) nach einem der vorhergehenden Ansprüche, **gekennzeichnet durch** einen von der Kontrolleinheit (6,6') ansteuerbaren Gaseinlass (21) zum Einlassen von Gas in den Probenraum (19).

7. Mikroplatten-Reader (1) nach einem der vorhergehenden Ansprüche, **dadurch gekennzeichnet, dass** die Kontrolleinheit (6,6') Gassensoren zum Messen von unterschiedlichen Gasen in dem Probenraum (19) und zur Steuerung der Zusammensetzung der Gasatmosphäre (7) um die Proben enthaltenden Wells (3) von einer in diesen Mikroplatten-Reader (1) eingesetzten Mikroplatte (4) umfasst.

8. Mikroplatten-Reader (1) nach einem der vorhergehenden Ansprüche, **dadurch gekennzeichnet, dass** die Kontrolleinheit (6,6') im Probenraum (19) einen Feuchtigkeitssensor zum Messen der Feuchtigkeit der Gasatmosphäre (7) und einen Temperatursensor zum Messen der Temperatur der Gasatmosphäre (7) umfasst.

9. Mikroplatten-Reader (1) nach einem der vorhergehenden Ansprüche, **dadurch gekennzeichnet, dass** er eine zweite Messeinrichtung (2") mit einer zweiten faseroptischen Leitung (33") und einem zweiten Wellenlängen selektierenden Monochromator (35") zum Messen der Fluoreszenz umfasst.

10. Mikroplatten-Reader (1) nach Anspruch 9, **dadurch gekennzeichnet, dass** die zweite Messeinrichtung (2") eine Fotomultiplier-Röhre (24) und einen zweiten Faserschieber (34") umfasst.

11. Mikroplatten-Reader (1) nach einem der vorhergehenden Ansprüche, **dadurch gekennzeichnet, dass** der Mikroplatten-Reader (1) die erste, zweite und dritte Messeinrichtung (2',2",2"') umfasst.

12. Mikroplatten-Reader (1) nach einem der vorhergehenden Ansprüche, **dadurch gekennzeichnet, dass** die erste, zweite bzw. dritte Messeinrichtung (2',2'',2''') eine erste, zweite bzw. dritte Optik (23',23",23"') umfasst, wobei zumindest eine dieser Optiken und/oder die Aufnahmeeinrichtung (5) bzw. eine eingesetzte Mikroplatte (4) in Richtung einer Z-Achse des kartesischen Koordinatensystems relativ zueinander bewegbar ausgebildet sind.

13. Mikroplatten-Reader (1) nach einem der vorhergehenden Ansprüche, **gekennzeichnet durch** eine Injektorvorrichtung (10), die zum Zugeben eines Reagens zu den Proben in Wells (3) einer in den Mikroplatten-Reader (1) eingesetzten Mikroplatte (4) ausgebildet ist.

14. Mikroplatten-Reader (1) nach einem der vorhergehenden Ansprüche, **dadurch gekennzeichnet, dass** er zum Messen der Absorbance, Fluoreszenz und/oder Lumineszenz mittels Top Reading eine erste oder zweite, oberhalb der Mikroplatte (4) angeordnete Optik (23',23") und/oder mittels Bottom Reading eine zweite oder dritte, unterhalb der Mikroplatte (4) angeordnete Optik (23",23"') umfasst.

15. Verfahren zum Messen lebender Zellen in einem Mikroplatten-Reader (1), der ein einen Innenraum (16) umgebendes Gehäuse (17) umfasst, **dadurch gekennzeichnet, dass** das Verfahren die folgenden Schritte umfasst:
a) Bereitstellen eines Mikroplatten-Readers (1) nach einem der Ansprüche 1 bis 14;
b) Aufnehmen von zumindest einer Mikroplatte (4) mit Proben enthaltenden Wells (3) in der Aufnahmeeinrichtung (5) dieses Mikroplatten-Readers (1);
c) Positionieren der Proben enthaltenden Wells (3) dieser Mikroplatte (4) gegenüber zumindest einer Messeinrichtung (2',2",2"') dieses Mikroplatten-Readers (1) mit der Aufnahmeeinrichtung (5);
d) Steuern der Zusammensetzung der Gasatmosphäre (7) in dem Probenraum (19); und
e) Verwenden dieser zumindest einen Messeinrichtung (2',2'',2''') zum Detektieren von Licht,
- das von Proben in Wells (3) einer in diesen Mikroplatten-Reader (1) eingesetzten Mikroplatte (4) ausgesendet wird, und/oder
- das von mit Licht durchstrahlten Proben in Wells (3) einer in diesen Mikroplatten-Reader (1) eingesetzten Mikroplatte (4) beeinflusst wird,
wobei die Konzentration zumindest eines Gases mit den im Probenraum (19) angeordneten Gassensoren der Kontrolleinheit (6,6') gemessen wird.

16. Verfahren nach Anspruch 15, ferner **gekennzeichnet durch** zumindest einen oder mehrere der folgenden Schritte:
f) Messen von Licht, das von mit Licht durchstrahlten Proben in Wells (3) einer in den Mikroplatten-Reader (1) eingesetzten Mikroplatte (4) beeinflusst wird, zum Messen der Absorbance der Probe,
g) Messen von Licht, das von mit Licht angestrahlten Proben in Wells (3) einer in den Mikroplatten-Reader (1) eingesetzten Mikroplatte (4) ausgesendet wird, zum Messen der Fluoreszenz der Probe, und
h) Messen von Licht, das von Proben in Wells (3) einer in den Mikroplatten-Reader (1) eingesetzten Mikroplatte (4) ausgesendet wird, zum Messen der Lumineszenz der Probe.

17. Verfahren nach Anspruch 16, **dadurch gekennzeichnet, dass** in dem Probenraum (19) eine definierte Gasatmosphäre (7) eingestellt wird und mit der eingestellten Gasatmosphäre (7) des Schrittes d) zumindest einer oder mehrere der Schritte f), g) und h) ausgeführt wird.

18. Verfahren nach Anspruch 17, **gekennzeichnet durch** Steuern des Anteils in der Gasatmosphäre (7) im Probenraum (19) von mindestens einem bestimmten Gas aus unterschiedlichen Gasen, das ausgewählt ist aus einer Gruppe, die Stickstoff, Kohlendioxid, Sauerstoff, Kohlenmonoxid, Schwefelwasserstoff und/oder Schwefeldioxid umfasst.

19. Verfahren nach einem der Ansprüche 17 oder 18, **gekennzeichnet durch** Steuern der Feuchtigkeit und/oder der Temperatur der Gasatmosphäre (7) in dem Probenraum (19) mittels der Kontrolleinheit (6,6').

20. Verfahren nach einem der Ansprüche 18 oder 19, **gekennzeichnet durch** Zugeben eines Reagens zu mindestens einer Probe in mindestens einem Well (3) der Mikroplatte (4) mittels einer Injektorvorrichtung (10) und **dadurch** Bewirken einer mittels zumindest einer der Messeinrichtungen (2',2'',2''') detektierbaren chemischen Reaktion.

21. Verfahren nach einem der Ansprüche 17 bis 20, **dadurch gekennzeichnet, dass** die Kontrolleinheit (6, 6') einen O₂-Sensor und/oder einen CO₂-Sensor zur Steuerung des Sauerstoff- und/oder Kohlendioxidgehalts der Gasatmosphäre (7) um die die Proben enthaltenden Wells (3) dieser in diesen Mikroplatten-Reader (1) eingesetzten zumindest einer Mikroplatte (4) umfasst, wobei die CO₂-bzw. O₂-Konzentration dieser Gasatmosphäre beim Vermessen mikroaerophiler, fakultativ anaerober oder obligat anaerober Mikroorganismen, Pilzen oder eukaryotischer Zellen durch gezieltes Einlassen von Kohlendioxid und/oder Stickstoff auf einem definierten Wert gehalten wird.

22. Verwendung eines Mikroplatten-Readers (1) gemäss einem der Ansprüche 1 bis 14 oder eines Verfahrens zum Messen lebender Zellen in einem Mikroplatten-Reader (1) gemäss einem der Ansprüche 15 bis 21, bei dem lebende Zellen in einer kontrollierten Gasatmosphäre (7) gemessen werden, wobei die lebenden Zellen ausgewählt sind aus einer Gruppe, die mikroaerophile, fakultativ anaerobe und obligat anaerobe Mikroorganismen sowie Pilze und eukaryotische Zellen umfasst.

## Claims

1. Microplate-reader (1), comprising:
a) at least one measuring device (2',2'',2'''), selected from a group comprising:
a1) a first measuring device (2') designed for measuring the absorbance of samples that are transilluminated with a first illumination device (11') in wells (3) of a microplate (4) inserted in the microplate-reader (1),
a2) a second measuring device (2") designed for measuring the fluorescence of samples that are irradiated with light of a first illumination device (11') in wells (3) of a microplate (4) inserted in the microplate-reader (1), and
a3) a third measuring device (2"') designed for measuring the luminescence of samples in wells (3) of a microplate (4) inserted in the microplate-reader (1);
b) a holding device (5) designed for accommodating of at least one microplate (4) during measuring with at least one of the measuring devices (2',2'',2''');
c) a zoning means alternatively configured as a separating plate (15) or an interior housing (39), which subdivides an interior space (16) of a housing (17) of the microplate-reader (1) into a sample compartment (19) comprising a gas atmosphere (7) surrounding the samples-containing wells (3) of an inserted microplate (4), and an appliance compartment (18), wherein the sample compartment (19) being separated from the appliance compartment substantially gas-tight, and wherein the zoning means being stationary located inside the housing (17) and comprising at least one opening (20) which is designed such that light which is irradiated or influenced by samples in wells (3) of a microplate (4) inserted in the microplate-reader (1) can pass there through from the sample compartment (19) to the appliance compartment (18); and
d) a control unit (6,6') for controlling the composition of a gas atmosphere (7) in the sample compartment (19), the control unit (6,6') comprising gas sensors located in the sample compartment (19) for measuring at least one gas of the gas atmosphere (7) in the in the sample compartment (19);
**characterized in that** the microplate-reader (1) further comprises at least one first illumination device (11') with a flash lamp (36), a first fiber-optical line (33'), and a first wavelength-selective monochromator (35'), the first illumination device (11') being configured for transilluminating or irradiating of samples in wells (3) of a microplate (4) inserted in the microplate-reader (1) with light of selected wavelengths, the first illumination device (11') of the microplate-reader (1) comprising at least a first optical system (23') located in the opening (20) of the separating plate (15) or in a wall of the interior housing (39);
and **in that** the holding device (5) is further configured for positioning the samples-containing wells (3) of this at least one microplate (4) relative to the at least one measuring device (2',2'',2''') at least in the direction of an axis of a Cartesian coordinate system and entirely is located in the sample compartment (19) and surrounded by the gas atmosphere of the sample compartment (19).

2. Microplate-reader (1) according to claim 1, **characterized in that** the sample compartment (19) is separated from the appliance compartment (18) substantially light-tight by means of the separating plate (15) or the interior housing, wherein a proofing device extending around the opening (20) being provided for light-tight and/or gas-tight separation.

3. Microplate-reader (1) according to claim 1 or 2, **characterized in that** a movement device for mixing and/or circulating gas that is present in and/or flowing into the sample compartment (19) is arranged in the sample compartment (19).

4. Microplate-reader (1) according to claim 3, **characterized in that** the movement device comprises at least one of the following devices: a blower (22), a device comprising one or more baffle plates, an agitation device comprising one or more paddles, or a structured jet nozzle system.

5. Microplate-reader (1) according to one of the claims 1 to 4, **characterized in that** the control unit (6,6') comprises a computer (28) that comprises corresponding software, wherein said computer (28) is configured to be connectable to a central computer (29) of the microplate-reader (1) or is integrated therein.

6. Microplate-reader (1) according to one of the preceding claims, **characterized by** a gas inlet (21) which is configured to be actuatable by the control unit (6,6') and for admitting gas into the sample compartment (19).

7. Microplate-reader (1) according to one of the preceding claims, **characterized in that** the control unit (6,6') comprises gas sensors for measuring different gases in the sample compartment (19) and for controlling the composition of the gas atmosphere (7) surrounding the samples-containing wells (3) of a microplate (4) inserted in this microplate-reader (1).

8. Microplate-reader (1) according to one of the preceding claims, **characterized in that** the control unit (6,6') comprises, in the sample compartment (19), a humidity sensor for measuring the humidity of the gas atmosphere (7) and a temperature sensor for measuring the temperature of the gas atmosphere (7).

9. Microplate-reader (1) according to one of the preceding claims, **characterized in that** it comprises a second measuring device (2") with a second fiber-optical line (33") and a second wavelength-selective monochromator (35") for measuring the fluorescence.

10. Microplate-reader (1) according to claim 9, **characterized in that** the second measuring device (2") comprises a photomultiplier tube (24) and a second fiber slide (34").

11. Microplate-reader (1) according to one of the preceding claims, **characterized in that** the microplate-reader (1) comprises the first, second, and third measuring device (2',2'',2''').

12. Microplate-reader (1) according to one of the preceding claims, **characterized in that** the first, second, or third measuring device (2',2",2"') comprises a first, second, or third optical system (23',23'',23'''), wherein at least one of these optical systems and/or the holding device (5) and/or an inserted microplate (4) are configured to be movable relative to each other in the direction of a Z-axis of the Cartesian coordinate system.

13. Microplate-reader (1) according to one of the preceding claims, **characterized by** an injector apparatus (10) configured for adding a reagent to the samples in wells (3) of a microplate (4) inserted in the microplate-reader (1).

14. Microplate-reader (1) according to one of the preceding claims, **characterized in that** it comprises, for measuring the absorbance, fluorescence and/or luminescence utilizing Top Reading, a first or second optical system (23',23") located above the microplate (4), and/or utilizing Bottom Reading, a second or third optical system (23",23"') located beneath the microplate (4).

15. Method for measuring living cells in a microplate-reader (1) comprising a housing (17) surrounding an interior space (16), **characterized in that** the method comprises the following steps:
a) providing a microplate-reader (1) according to one of the claims 1 to 14;
b) accommodating in the holding device (5) of this microplate-reader (1) at least one microplate (4) comprising samples-containing wells (3);
c) positioning the samples-containing wells (3) of this microplate (4) in relation to at least one measuring device (2',2'',2''') of this microplate-reader (1) using the holding device (5);
d) controlling the composition of the gas atmosphere (7) in the sample compartment (19); and
e) using the at least one measuring device (2',2",2"') for detecting light,
- which is emitted by samples in wells (3) of a microplate (4) inserted in this microplate-reader (1), and/or
- which is influenced by samples transilluminated by light in wells (3) of a microplate (4) inserted in this microplate-reader (1),
wherein the concentration of at least one gas is measured with the gas sensors of the control unit (6',6") located in the sample compartment (19).

16. Method according to claim 15, further **characterized by** at least one or more of the following steps:
f) measuring light, which is influenced by samples that are transilluminated by light in wells (3) of a microplate (4) inserted in the microplate-reader (1), for measuring the absorbance of the sample,
g) measuring light, which is emitted from samples that are irradiated by light in wells (3) of a microplate (4) inserted in the microplate-reader (1), for measuring the fluorescence of the sample, and
h) measuring light, which is emitted from samples in wells (3) of a microplate (4) inserted in the microplate-reader (1), for measuring the luminescence of the sample.

17. Method according to claim 16, **characterized in that** a defined gas atmosphere (7) is adjusted in the sample compartment (19) and at least one or more of the steps f), g) and h) is executed with the adjusted gas atmosphere (7) of the step d).

18. Method according to claim 17, **characterized by** controlling the proportion in the gas atmosphere (7) in the sample compartment (19) of at least one particular gas out of different gases, wherein the particular gas is selected from a group, which comprises nitrogen, carbon dioxide, oxygen, carbon monoxide, hydrogen sulphide and sulphur dioxide.

19. Method according to one of the claims 17 or 18, **characterized by** controlling the humidity and/or the temperature of the gas atmosphere (7) in the sample compartment (19) by means of the control unit (6,6').

20. Method according to one of the claims 18 or 19, **characterized by** adding a reagent to at least one sample in at least one well (3) of the microplate (4) by means of an injector apparatus (10), and thus causing a chemical reaction which can be detected by means of at least one of the measuring devices (2',2'',2''').

21. Method according to one of the claims 17 to 20, **characterized in that** the control unit (6,6') comprises an O₂ sensor and/or a CO₂ sensor for controlling the oxygen and/or carbon dioxide content of the gas atmosphere (7) surrounding the samples-containing wells (3) of the at least one microplate (4) inserted in this microplate-reader (1), wherein the CO₂ and/or the O₂ concentration of this gas atmosphere is held at a defined value by purposefully introducing carbon dioxide and/or nitrogen during the measurement of microaerophilic, optionally anaerobic or obligatorily anaerobic microorganisms, fungi or eukaryotic cells.

22. Utilizing a microplate-reader (1) according to one of the claims 1 to 14 or a method for measuring living cells in a microplate-reader (1) according to one of the claims 15 to 21, wherein living cells are measured in a controlled gas atmosphere (7), wherein the living cells are chosen from a group which comprises microaerophilic, optionally anaerobic and obligatorily anaerobic microorganisms as well as fungi and eukaryotic cells.

## Revendications

1. Lecteur de microplaques (1) comprenant :
a) au moins un dispositif de mesure (2',2'',2''') sélectionné à partir d'un groupe comprenant :
a1) d'un premier dispositif de mesure (2') conçu pour mesurer l'absorbance d'échantillons traversés par la lumière d'un premier dispositif d'éclairage (11') dans les puits (3) d'une microplaque (4) montée dans le lecteur de microplaques (1),
a2) d'un deuxième dispositif de mesure (2") conçu pour mesurer la fluorescence d'échantillons illuminés par la lumière d'un premier dispositif d'éclairage (11') dans les puits (3) d'une microplaque (4) montée dans le lecteur de microplaques (1), et
a3) d'un troisième dispositif de mesure (2"') conçu pour mesurer la luminescence d'échantillons dans les puits (3) d'une microplaque (4) montée dans le lecteur de microplaques (1) ;
b) un dispositif de réception (5) conçu pour recevoir au moins une microplaque (4) pendant la mesure avec au moins l'un des dispositifs de mesure (2',2'',2''') ;
c) un moyen de limitation, sous la forme d'une plaque de séparation (15) ou d'un boîtier intérieur (39) lequel moyen divise un espace intérieur (16) d'un boîtier (17) du lecteur de microplaques (1) en un compartiment à échantillons (19) avec une atmosphère gazeuse (7) entourant les puits (3) contenant les échantillons d'une microplaque (4) utilisée, et en un compartiment d'appareil (18), le compartiment à échantillons (19) étant séparé du compartiment d'appareil essentiellement de façon étanche au gaz, et le moyen de limitation étant agencé de façon fixe dans le boîtier (17) et comprenant au moins une ouverture (20) qui est réalisée de telle façon que la lumière émise ou influencée par les échantillons dans les puits (3) d'une microplaque (4) montée dans le lecteur de microplaques (4) peut passer du compartiment à échantillons (19) au compartiment d'appareil (18) ; et
d) une unité de contrôle (6,6') pour régler la composition d'une atmosphère gazeuse (7) dans le compartiment à échantillons (19), l'unité de contrôle (6,6') comprenant des capteurs de gaz agencés dans le compartiment à échantillons (19) pour mesurer la concentration d'au moins un gaz de l'atmosphère gazeuse (7) dans le compartiment à échantillons (19) ;
**caractérisé en ce que** le lecteur de microplaques (1) comprend en outre au moins un premier dispositif d'éclairage (11') avec une lampe flash (36), une première conduite à fibre optique (33') et un premier monochromateur (35') sélectionnant les longueurs d'onde, ce premier dispositif d'éclairage (11') étant conçu pour traverser ou illuminer les échantillons dans les puits (3) d'une microplaque (4) montée dans le lecteur de microplaques (1) avec une lumière de longueurs d'ondes choisies, le premier dispositif d'éclairage (11') du lecteur de microplaques (1) comprenant au moins une première optique (23') qui est agencée dans l'ouverture (20) dans la plaque de séparation (15) ou dans une paroi du boîtier intérieur (39) ;
et **en ce que** le dispositif de réception (5) est en outre conçu mobile pour positionner les puits (3) contenant les échantillons de cette au moins une microplaque (4) par rapport à au moins un dispositif de mesure (2',2'',2''') au moins dans la direction d'un axe d'un système de coordonnées cartésien et est totalement agencé dans le compartiment à échantillons (19) et est entouré de l'atmosphère gazeuse du compartiment à échantillons (19).

2. Lecteur de microplaques (1) selon la revendication 1, **caractérisé en ce que** le compartiment à échantillons (19) est en outre séparé essentiellement de façon étanche à la lumière du compartiment d'appareil (18) par la plaque de séparation (15) ou le boîtier intérieur, dans lequel il est prévu de préférence pour la séparation étanche à la lumière et/ou étanche au gaz un dispositif d'étanchéité s'étendant tout autour de l'ouverture (20).

3. Lecteur de microplaques (1) selon la revendication 1 ou 2, **caractérisé en ce qu'**il est agencé dans le compartiment à échantillons (19) un dispositif de déplacement pour mélanger et/ou brasser le gaz présent et/ou affluant dans le compartiment à échantillons (19).

4. Lecteur de microplaques (1) selon la revendication 3, **caractérisé en ce que** le dispositif de déplacement comprend au moins l'un des dispositifs suivants :
un ventilateur (22), un dispositif avec une ou plusieurs plaques de déviation, un agitateur avec un ou plusieurs palettes ou un système de buses structuré.

5. Lecteur de microplaques (1) selon l'une des revendications 1 à 4, **caractérisé en ce que** l'unité de contrôle (6,6') comprend un ordinateur (28) avec des logiciels correspondants, l'ordinateur (28) pouvant être raccordé à un ordinateur central (29) du lecteur de microplaques (1) ou bien étant intégré à ce dernier.

6. Lecteur de microplaques (1) selon l'une des revendications précédentes, **caractérisé par** une entrée de gaz (21) pouvant être commandée par l'unité de contrôle (6,6') pour laisser entrer du gaz dans le compartiment à échantillons (19).

7. Lecteur de microplaques (1) selon l'une des revendications précédentes, **caractérisé en ce que** l'unité de contrôle (6,6') comprend des capteurs de gaz pour mesurer les différents gaz présents dans le compartiment à échantillons (19) et pour régler la composition de l'atmosphère gazeuse (7) autour des puits (3) contenant les échantillons d'une microplaque (4) montée dans ce lecteur de microplaques (1).

8. Lecteur de microplaques (1) selon l'une des revendications précédentes, **caractérisé en ce que** l'unité de contrôle (6,6') comprend dans le compartiment à échantillons (19) un détecteur d'humidité pour mesurer l'humidité de l'atmosphère gazeuse (7) et un capteur de température pour mesurer la température de l'atmosphère gazeuse (7).

9. Lecteur de microplaques (1) selon l'une des revendications précédentes, **caractérisé en ce qu'**il comprend un deuxième dispositif de mesure (2") avec une deuxième conduite à fibre optique (33") et un deuxième monochromateur sélectionnant les longueurs d'ondes (35") pour mesurer la fluorescence.

10. Lecteur de microplaques (1) selon la revendication 9, **caractérisé en ce que** le deuxième dispositif de mesure (2") comprend un tube photomultiplicateur (24) et un deuxième volet à fibre (34").

11. Lecteur de microplaques (1) selon l'une des revendications précédentes, **caractérisé en ce que** le lecteur de microplaques (1) comprend le premier, le deuxième et le troisième dispositif de mesure (2',2'',2''').

12. Lecteur de microplaques (1) selon l'une des revendications précédentes, **caractérisé en ce que** le premier, le deuxième et le troisième dispositif de mesure (2',2'',2''') comprend une première, une deuxième et une troisième optique (23',23'',23'''), au moins l'une de ces optiques et/ou le dispositif de réception (5) ou une microplaque utilisée (4) étant réalisés mobiles les uns par rapport aux autres dans le sens de l'axe Z du système de coordonnées cartésien.

13. Lecteur de microplaques (1) selon l'une des revendications précédentes, **caractérisé par** un dispositif d'injection (10) qui est conçu pour ajouter un réactif aux échantillons dans les puits (3) d'une microplaque (4) montée dans le lecteur de microplaques (1).

14. Lecteur de microplaques (1) selon l'une des revendications précédentes, **caractérisé en ce qu'**il comprend pour mesurer l'absorbance, la fluorescence et/ou la luminescence au moyen d'une lecture supérieure *(Top Reading)* une première ou une deuxième optique (23',23") agencée au-dessus de la microplaque (4) et/ou au moyen d'une lecture inférieure (*Bottom Reading*) une deuxième ou une troisième optique (23'',23''') agencée en-dessous de la microplaque (4).

15. Procédé pour mesurer des cellules vivantes dans un lecteur de microplaques (1) qui comprend un boîtier (17) entourant un espace intérieur (16), **caractérisé par** les étapes comprenant :
a) préparer un lecteur de microplaques (1) selon l'une des revendications 1 à 14 ;
b) recevoir au moins une microplaque (4) avec des puits (3) contenant des échantillons dans le dispositif de réception (5) de ce lecteur de microplaques (1) ;
c) positionner les puits (3) contenant les échantillons de cette microplaque (4) par rapport à au moins un dispositif de mesure (2',2'',2''') de ce lecteur de microplaques (1) avec le dispositif de réception (5) ;
d) régler la composition de l'atmosphère gazeuse (7) dans le compartiment à échantillons (19) ; et
e) utiliser ce au moins un dispositif de mesure (2',2'',2''') pour détecter la lumière,
- qui est émise par les échantillons dans les puits (3) d'une microplaque (4) montée dans ce lecteur de microplaques (1), et/ou
- qui est influencée par les échantillons irradiés par la lumière dans les puits (3) d'une microplaque (4) montée dans ce lecteur de microplaques (1),
la concentration d'au moins un gaz étant mesurée avec les capteurs de gaz de l'unité de contrôle (6,6') agencés dans le compartiment à échantillons (19).

16. Procédé selon la revendication 15, **caractérisé en outre par** au moins une ou plusieurs des étapes suivantes consistant à :
f) mesurer la lumière qui est influencée par les échantillons traversés par la lumière dans les puits (3) d'une microplaque (4) montée dans le lecteur de microplaques (1), pour mesurer l'absorbance de l'échantillon,
g) mesurer la lumière qui est irradiés par les échantillons illuminés par la lumière dans les puits (3) d'une microplaque (4) montée dans le lecteur de microplaques (1), pour mesurer la fluorescence de l'échantillon, et
h) mesurer la lumière qui est émise par les échantillons dans les puits (3) d'une microplaque (4) montée dans le lecteur de microplaques (1), pour mesurer la luminescence de l'échantillon.

17. Procédé selon la revendication 16, **caractérisé en ce qu'**il est réglé dans le compartiment à échantillons (19) une atmosphère gazeuse (7) définie et **en ce qu'**on réalise avec l'atmosphère gazeuse (7) déterminée à l'étape d) au moins une ou plusieurs des étapes f), g) et h).

18. Procédé selon la revendication 17, **caractérisé par** le réglage de la part de l'atmosphère gazeuse (7) dans le compartiment à échantillons (19) d'au moins un certain gaz à partir de différents gaz choisis à partir d'un groupe qui comprend de l'azote, du dioxyde de carbone, de l'oxygène, du monoxyde de carbone, de l'acide sulfhydrique et/ou du dioxyde de soufre.

19. Procédé selon l'une des revendications 17 ou 18, **caractérisé par** le réglage de l'humidité et/ou de la température de l'atmosphère gazeuse (7) dans le compartiment à échantillons (19) au moyen de l'unité de contrôle (6,6').

20. Procédé selon l'une des revendications 18 ou 19, **caractérisé par** l'ajout d'un réactif à au moins un échantillon dans au moins un puits (3) de la microplaque (4) au moyen d'un dispositif d'injection (10) et par l'induction d'une réaction chimique pouvant être détectée par au moins un des dispositifs de mesure (2',2'',2''').

21. Procédé selon l'une des revendications 17 à 20, **caractérisé en ce que** l'unité de contrôle (6,6') comprend un capteur de O₂ et/ou un capteur de CO₂ pour régler la teneur en oxygène et/ou en dioxyde de carbone de l'atmosphère gazeuse (7) autour des puits (3) contenant les échantillons de cette au moins une microplaque (4) montée dans le lecteur de microplaques (1), la concentration de CO₂ ou de O₂ de cette atmosphère gazeuse étant maintenue à une valeur définie par une admission ciblée de dioxyde de carbone et/ou d'azote en mesurant des microorganismes microaérophiles, anaérobies facultatifs ou anaérobies obligatoires, des champignons ou des cellules eucaryotes.

22. Utilisation d'un lecteur de microplaques (1) selon l'une des revendications 1 à 14 ou d'un procédé pour mesurer des cellules vivantes dans un lecteur de microplaques (1) selon l'une des revendications 15 à 21, dans lequel les cellules vivantes sont mesurées dans une atmosphère gazeuse (7) contrôlée, les cellules vivantes étant choisies à partir d'un groupe comprenant les microorganismes microaérophiles, anaérobies facultatifs et anaérobies obligatoires, ainsi que les champignons et les cellules eucaryotes.
